# EUROPEAN PATENT SPECIFICATION

(11) **EP 2 798 089 B1**
(45) Date of publication and mention of the grant of the patent: **23.05.2018**
(21) Application number: 12863528.1
(22) Date of filing: 21.12.2012
(51) Int. Cl.: C12Q 1/68

(54) **METHODS AND COMPOSITIONS FOR PERFORMING NUCLEIC ACID AMPLIFICATION REACTIONS**
VERFAHREN UND ZUSAMMENSETZUNGEN ZUR DURCHFÜHRUNG VON NUCLEINSÄUREAMPLIFIKATIONSREAKTIONEN
PROCÉDÉS ET COMPOSITIONS POUR LA MISE EN UVRE DE RÉACTIONS D'AMPLIFICATION D'ACIDE NUCLÉIQUE

(30) Priority: 30.12.2011 US 201161582120 P
(43) Date of publication of application: 05.11.2014
(73) Proprietor: Bio-rad Laboratories, Inc., Hercules, CA 94547 (US)
(72) Inventor: MONTESCLAROS, Luz, Pittsburg, CA 94565 (US); WYATT, Paul, Pleasanton, CA 94566 (US)
(74) Representative: Avidity IP
(86) International application number: PCT/US2012/071517
(87) International publication number: WO 2013/101783

(56) References cited:
- WO-A1-2009/091719
- WO-A2-2005/118875
- US-A1- 2003 027 196
- US-A1- 2009 298 052
- US-A1- 2010 279 305
- WARREN LUIGI ET AL: "Transcription factor profiling in individual hematopoietic progenitors by digital RT-PCR", PROCEEDINGS OF THE NATIONAL ACADEMY OF SCIENCES, NATIONAL ACADEMY OF SCIENCES, US, vol. 103, no. 47, 1 November 2006 (2006-11-01), pages 17807-17812, XP002479982, ISSN: 0027-8424, DOI: 10.1073/PNAS.0608512103
- BEER ET AL.: 'On-Chip Single-Copy Real-Time Reverse-Transcription PCR in Isolated Picoliter Droplets.' ANALYTICAL CHEMISTRY vol. 80, no. 6, 15 March 2008, pages 1854 - 1858, XP002575963

## Description

### BACKGROUND

The ability to amplify ribonucleic acid (RNA) is an important aspect of efforts to elucidate biological processes. Total cellular mRNA represents gene expression activity at a defined time. Gene expression is affected by cell cycle progression, developmental regulation, or response to internal and external stimuli. The profile of expressed genes for any cell type in an organism reflects normal or disease states, response to various stimuli, developmental stages, or cell differentiation. Non-coding RNAs have been shown to be of great importance in regulation of various cellular functions and in certain disease pathologies. Such RNAs are often present in very low levels. Thus, amplification methods capable of amplifying low abundance RNAs are of great importance.

A number of methods for the analysis of gene expression have been described. See, for example, U.S. Pat. Nos. 6,251,639, 6,692,918, 6,686,156, 5,744,308; 6,143,495; 5,824,517; 5,829,547; 5,888,779; 5,545,522; 5,716,785; 5,409,818; EP 0971039A2; EP0878553A2; and U.S. published patent applications nos. 2002/0115088, 2003/0186234, 2003/0087251, and 2004/0023271. These include quantification of specific mRNAs, and the simultaneous quantification of a large number of mRNAs, as well as the detection and quantification of patterns of expression of known and unknown genes.

Luigi et al., Proc. National Academy of Sceinces, 103, 17807-17812 (2006) discloses analysis of transcription factor expression employing a microfluidic chip assay.

RNA amplification can be performed using the reverse transcriptase-polymerase chain reaction (RT-PCR) method and variations thereof. These methods are based on replication of RNA by reverse transcriptase to form single stranded DNA complementary to the RNA (cDNA), which is followed by polymerase chain reaction (PCR) amplification to produce multiple copies of double stranded DNA.

### SUMMARY

Described is a method for reverse transcribing and amplifying an RNA, the method comprising: (a) contacting an RNA with a reaction mixture comprising: (i) a first enzyme comprising reverse transcriptase activity, (ii) manganese, and (iii) magnesium, under a condition suitable for reverse transcription reaction, thereby reverse transcribing the RNA into a DNA, wherein the magnesium is inaccessible to the first enzyme during at least part of the duration of the reverse transcription reaction; and (b) amplifying the DNA in the reaction mixture with a second enzyme having DNA-dependent DNA polymerase activity, wherein the magnesium is accessible to the second enzyme during the amplification reaction.

In some cases, the magnesium is inaccessible to the first enzyme during the initiation of the reverse transcription reaction. In some cases, the reaction mixture contains a chelator. In some cases, the chelator is EDTA or EGTA. In some cases, the reaction mixture does not contain a chelator.

In some cases, the first enzyme comprises a thermostable reverse transcriptase. In some cases, the second enzyme comprises a hot-start DNA polymerase. In some cases, the first enzyme and the second enzyme are the same enzyme.

In some cases, the reaction mixture comprises a manganese salt selected from the group consisting of MnCl₂, MnSO₄, and Mn(OAc)₂. In some cases, the concentration of the manganese in the reaction mixture is from 0.5 mM to 5mM. In some cases, the concentration of the manganese in the reaction mixture is about 1 mM.

In some cases, the reaction mixture comprises a magnesium salt selected from the group consisting of MgCl₂, MgSO₄, and Mg(OAc)₂. In some cases, the molar ratio between the magnesium and the dNTPs presented in the reaction mixture at the beginning of the step (a) is about 1:1. In some cases, the molar ratio between the magnesium and the total amount of the chelator and the dNTPs present in the reaction mixture at the beginning of the step (a) is about 1:1. In some cases, the concentration of the magnesium in the reaction mixture is about 1 mM. In some cases, a ratio between the manganese and the magnesium in the reaction mixture is from about 1:4 to about 3:1. In some cases, a ratio between the manganese and the magnesium in the reaction mixture is about 1.

In some cases, the step (a) is carried out at about 55 °C to about 60 °C. In some cases, step (b) is carried out by thermocycling. In some cases, the method comprises a digital PCR reaction. In some cases, the digital PCR reaction is a droplet digital PCR reaction. In some cases, the step (a) and the step (b) are carried out in the same droplet. In some cases, there is no additional manipulation of the droplet between the step (a) and the step (b).

In some cases, the droplet has a volume that is between about 1 pL and about 100 nL. In some cases, the step (a) and/or (b) is carried out in a droplet having a volume that is between about 1 pL and about 100 nL. In some cases, the step (a) and/or (b) is carried out in a plurality of droplets, wherein each droplet has a volume that is between about 1 pL and about 100 nL.

In another aspect, a method for preparing a reaction mixture is provided, the method comprising: (a) providing a first solution comprising: (i) a buffer; (ii) a monovalent cation; (iii) a magnesium salt; (iv) dATP, dCTP, dGTP, and dTTP, or analogs thereof; (v) a first enzyme comprising reverse transcriptase activity; (vi) a second enzyme comprising DNA-dependent DNA polymerase activity; (vii) an oligonucleotide to initiate the reverse transcription of the RNA to the cDNA; and (viii) a pair of primers for amplifying a target sequence, wherein the first solution does not contain manganese; and (b) mixing the first solution with a second solution comprising manganese.

In some cases, the first solution comprises a magnesium salt selected from the group consisting of MgCl₂, MgSO₄, and Mg(OAc)₂. In some cases, the first solution comprises a dNTP analogue. In some cases,
the first enzyme comprises a thermostable reverse transcriptase. In some cases, the second enzyme comprises a hot-start DNA polymerase. In some cases, the monovalent cation is K⁺ or Na⁺.

In some cases, the second solution comprises a manganese salt selected from the group consisting of MnCl₂, MnSO₄, and Mn(OAc)₂.

In another aspect, a method for reverse transcribing and amplifying an RNA is provided, the method comprising: (a) contacting an RNA comprising the target sequence with a reaction mixture prepared according any method described above, thereby reverse transcribing the RNA into a DNA; and (b) amplifying the DNA in the reaction mixture with the second enzyme. In some cases, the reverse transcription and amplification reactions are carried out in a droplet having a volume that is between about 1 pL and about 100 nL. In some cases, the magnesium is inaccessible to the first enzyme during at least part of the duration of the reverse transcription reaction. In some cases, the magnesium is accessible to the second enzyme during the amplification reaction.

In another aspect, a kit for reverse transcribing and amplifying an RNA is provided, the kit comprising a first solution comprising: (i) a buffer; (ii) a monovalent cation; (iii) a magnesium salt, and optionally a chelator; (iv) dATP, dCTP, dGTP, and dTTP, or analogs thereof; (v) a first enzyme comprising reverse transcriptase activity; and (vi) a second enzyme comprising DNA-dependent DNA polymerase activity; wherein the reaction mixture does not contain manganese, and wherein the molar ratio between (1) the magnesium and (2) the total amount of (a) the chelator, if present, and (b) the dNTPs present, in the first solution is about 1.

In some cases, the first enzyme and the second enzyme are the same enzyme. In some cases, the first solution comprises a magnesium salt selected from the group consisting of MgCl₂, MgSO₄, and Mg(OAc)₂. In some cases, the first solution comprises a dNTP analogue. In some cases, the first enzyme comprises a thermostable reverse transcriptase. In some cases, the second enzyme comprises a hot-start DNA polymerase. In some cases, the monovalent cation is K⁺ or Na⁺. In some cases, the kit further comprises a second solution comprising a manganese salt. In some cases, the manganese salt is selected from the group consisting of MnCl₂, MnSO₄, and Mn(OAc)₂.

In another aspect, a method for reverse transcribing and amplifying an RNA in a single droplet is provided, the method comprising: (a) contacting an RNA with a reaction mixture comprising a first enzyme comprising reverse transcriptase activity, under a condition suitable for reverse transcription reaction, thereby reverse transcribing the RNA into a DNA; and (b) amplifying the DNA in the reaction mixture with a second enzyme having DNA-dependent DNA polymerase activity, wherein the reaction mixture is contained in a single droplet that does not contain a bead.

In some cases, the single droplet does not contain a gel. In some cases, the reaction mixture comprises manganese and magnesium. In some cases, the magnesium is inaccessible to the first enzyme during at least part of the duration of the reverse transcription reaction. In some cases, the magnesium is accessible to the second enzyme during the amplification reaction.

In some cases, the reverse transcription and the amplification reaction each independently has an efficiency of more than 60%. In some cases, the droplet has a volume about 1 pL to 100 nL. In some cases, the reaction mixture is contained in a plurality of droplets. In some cases, each of the plurality of droplets has a volume about 1 pL to about 100 nL.

In some cases, a first subpopulation of the plurality of droplets has a zero, one or more of a target RNA molecule per droplet. In some cases, there is no manipulation of the droplet between the step (a) and the step (b).

### BRIEF DESCRIPTION OF THE DRAWINGS

The novel features are set forth herein with particularity in the appended claims. A better understanding of the features and advantages will be obtained by reference to the following detailed description that sets forth illustrative embodiments, in which the principles described herein are utilized, and the accompanying drawings of which
**FIG. 1** illustrates that the ddPCR™ Hot-start 1 Step RT-PCR Master Mix has 3 log dynamic range and linear quantitation.
**FIG. 2A** and **FIG. 2B** illustrate that the ddPCR™ Hot-start 1 Step RT-PCR Master Mix has 3 log dynamic range and linear quantitation.
**FIG. 3** illustrates that there is no variation due to workflow stability with the ddPCR™ Hot-start 1 Step RT-PCR Master Mix.
**FIG. 4** illustrates the stability of RT droplets on ice prior to RT-PCR.

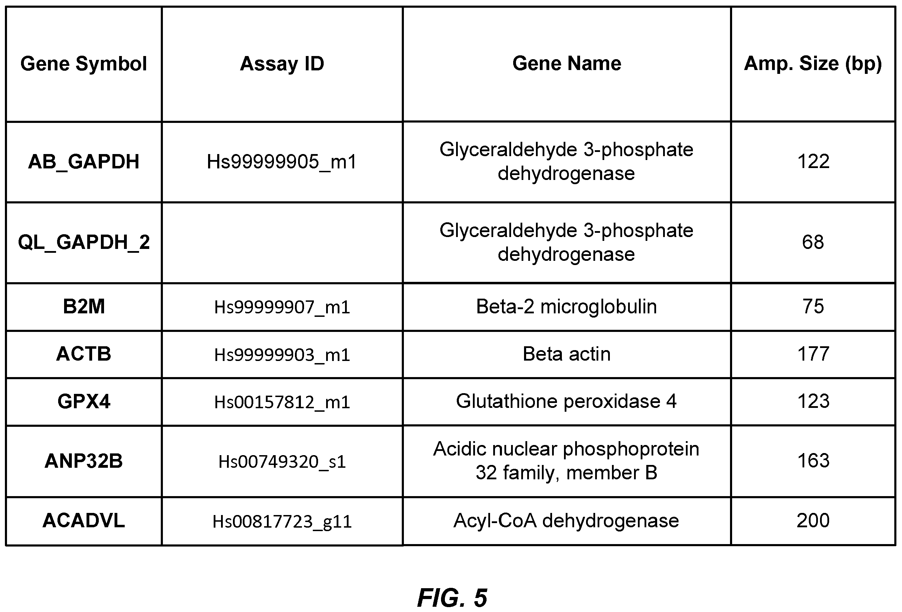

**FIG. 5** depicts a assay panel used in optimization studies.
**FIG. 6** depicts pH verification for different assays.
**FIG. 7** depicts pH verification for different assays.
**FIG. 8** depicts that sizes of RT Mix droplets are similar to that of a control ddPCR Master Mix.

### DETAILED DESCRIPTION

The present invention provides a method of performing reverse transcription and amplification, the method comprising: (a) providing a first solution including (i) a buffer (ii) a magnesium salt,
(iii) dNTPs comprising dATP, dCTP, dGTP, and dTTP, or analogs thereof,
(iv) an enzyme comprising reverse transcriptase activity, and
(v) an enzyme comprising DNA-dependent DNA polymerase activity;
   (b) mixing the first solution with a second solution comprising manganese;
   (c) forming emulsion droplets containing the first solution mixed with the second solution;
   (d) reverse-transcribing RNA in the droplets with the first enzyme to form DNA; and
   (e) amplifiying a target sequence of the DNA in the emulsion droplets with the second enzyme;
   wherein the molar ratio between the magnesium and the dNTPs in the first solution is about 1:1.

The terminology used herein is for the purpose of describing particular embodiments only and is not intended to be limiting. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising".

"About" can mean within 1 or more than 1 standard deviation, per the practice in the art. Alternatively, "about" can mean a range of up to 20%, up to 10%, up to 5%, or up to 1% of a given value. Alternatively, e.g., with respect to biological systems or processes, the term "about" can mean within an order of magnitude, e.g., within 5-fold or within 2-fold of a value.

### I. Method for One-step RT- PCR

### Overview

Described herein are methods, compositions, and kits for carrying out an RT-PCR reaction in a single reaction mixture comprising two divalent cations, e.g., manganese and magnesium.

Generally, the total amount of sample RNA available can be limited by the amount of biological sample from which the RNA is derived. Biological samples can be limited in amount and can be of high value. Moreover, in some cases, the amount of the various RNA species in a sample is not equal; some RNA species can be more abundant than others, and the more abundant species can be easier to analyze. The ability to amplify RNA sequences can enable the analysis of less abundant, rare RNA species. The ability to analyze small samples, e.g., by means of nucleic acid amplification, can be advantageous for designing large scale screens of effector molecule libraries, for which reduction in sample volume can be a concern both for the ability to perform very large scale screening or ultra high throughput screening, and in view of the limiting amounts of library components. Methods of amplification from RNA templates have been described, for example, in U.S. Patent No. 6,946,251.

RT-PCR can be used to study the genomes of viruses whose genomes are composed of RNA, such as influenza virus A, and retroviruses such as HIV.

In some cases, RT-PCR can be used to measure gene expression levels Gene expression levels be used to determine the genotype or phenotype of an organism, to measure the health of an organism, or to measure a specific metabolic pathway.

RT-PCR can be carried out in two steps or one step. In a two-step RT-PCR scheme, the RNA can be first converted to cDNA in one reaction, e.g., using either a random hexamer to convert the total RNA to cDNA or a poly-dT primer for messenger RNA (mRNA) which contain a poly-A tail. In some cases, sequence specific primers are used. The cDNA can then be distributed for detection of specific sequences, e.g., using amplicon specific primers. The RT reaction and the PCR reaction can be carried out in two different reaction mixtures.

In some cases, a one step RT-PCR can be performed in a single reaction where the gene fragment of interested is converted to cDNA, and then the cDNA is amplified by PCR, all in the same reaction mixture.

The polymerase chain reaction (PCR) can be carried out using a thermostable DNA polymerase, such as Taq DNA polymerase, which can withstand the temperatures used to denature the amplified product in each cycle. PCR is well known in the art and has been described extensively in the scientific literature (see, for example, PCR Applications, 1999, (Innis et al., eds., Academic Press, San Diego), PCR Strategies, 1995, (Innis et al., eds., Academic Press, San Diego); PCR Protocols, 1990, (Innis et al., eds., Academic Press, San Diego); and PCR Technology, 1989, (Erlich, ed., Stockton Press, New York); each incorporated herein by reference).

Because reverse transcription using Taq DNA polymerase in a magnesium ion buffer can be inefficient, PCR amplification starting with an RNA template initially can be carried out by first reverse-transcribing the target RNA using, for example, a non-thermostable viral reverse transcriptase such as Molony Murine Leukemia Virus Reverse Transcriptase (MoMuLV RT) or AMV-RT, and then amplifying the resulting cDNA using a thermostable DNA polymerase.

A thermostable DNA polymerase can be used to efficiently reverse transcribe an RNA template by carrying out the reaction in a manganese buffer (Mn²⁺), rather than a magnesium (Mg²⁺) buffer, as can be preferred for primer extension using a DNA template. Efficient Mn²⁺-activated reverse transcription using a thermostable DNA polymerase is described, e.g., in U.S. Patent Nos. 5,310,652; 5,322,770; 5,407,800; 5,641,864; 5,561,058; and 5,693,517. As both the synthesis of cDNA from an RNA template and the synthesis of DNA from a DNA template can be carried out in a Mn²⁺ buffer, the use of a Mn²⁺ buffer can enable single-enzyme, coupled reverse transcription/amplification reactions.

The use of Mn²⁺ can increase the efficiency of reverse transcription but can also decrease the fidelity of DNA amplifications, which can result in an increased number of misincorporated nucleotides. Thus, single-enzyme, one-tube RNA amplification reactions using Mn²⁺ can suffer reduced fidelity in both the RT and DNA amplification phases of the reaction. Thus, when higher fidelity RNA amplification is desired, it can be preferable to carry out the reaction in two stages. This situation can be achieved by effectively removing the Mn²⁺ ions from the reverse-transcription mixture using a chelator, such as EDTA or EGTA, and then adding an appropriate Mg²⁺ containing DNA amplification mixture to complete the reaction. However, such a two-step RT-PCR reaction can be both cumbersome and time consuming.

Described herein are methods, compositions, and kits that allow both manganese and magnesium divalent cations to be added to a single reaction mixture that can be used to perform an RT-PCR reaction where the reverse transcription of the RNA to the cDNA is first performed at, e.g., about 55 to about 60°C, and the subsequent amplification reaction, e.g., PCR, can be performed using standard cycling conditions.

In one aspect, a method is provided herein for reverse transcribing and amplifying an RNA, the method comprising: (a) contacting an RNA with: (i) a first enzyme comprising reverse transcriptase activity, and (ii) a reaction mixture comprising manganese and magnesium, under a condition suitable for a reverse transcription reaction, thereby reverse transcribing the RNA into a DNA, wherein the magnesium is inaccessible to the first enzyme during at least part of the duration of the reverse transcription reaction; and (b) amplifying the DNA in the reaction mixture with a second enzyme having DNA-dependent DNA polymerase activity, wherein the magnesium is accessible to the second enzyme during the amplification reaction. In some cases, the RT reaction and amplification reaction are carried out in the same reaction mixture and there is no need of removing the manganese from the reaction mixture such as by adding a chelator after the RT reaction but prior to the amplification reaction.

### Reverse Transcriptase

In some cases, a first enzyme used in the provided methods and compositions comprises reverse transcriptase activity.

A reverse transcriptase can be an enzyme in a class of polymerases characterized as RNA-dependent DNA polymerases. In some cases, a reverse transcriptase can require a primer to synthesize DNA from an RNA template. A reverse transcriptase can be used to transcribe mRNA into cDNA, which can then be cloned into a vector for further manipulation. Examples of reverse transcriptases include HIV-1 reverse transcriptase, M-MLV (Moloney murine leukemia virus) reverse transcriptase, and AMV (avian myelobastosis virus) reverse transcriptase. In some cases, a reverse transcriptase can synthesize a DNA strand from single-stranded RNA, DNA, or an RNA:DNA hybrid.

In some cases, a reverse transcriptase comprises RNase H activity. In some cases, a reverse transcriptase lacks RNase H activity. In some cases, a reverse transcriptase has RNA-dependent DNA polymerase activity, ribonuclease H activity, and DNA-dependent DNA polymerase activity. In some cases, a reverse transcriptase has ribonuclease H activity and no DNA-dependent DNA polymerase activity. In some cases, a reverse transcriptase has DNA-dependent DNA polymerase activity and no ribonuclease H activity.

In some cases, a reverse transcriptase can generate a transcript of about, more than, less than, or at least 100, 500, 1000, 2000, 3000, 4000, 5000, 6000, 7000, 8000, 9000, 10,000, 11,000, 12,000, 13,000, 14,000, 15,000, 16,000, 17,000, 18,000, 19,000, or 20,000 bases. In some cases, a reverse transcriptase can generate a transcript of about 0.5 kb to 20 kb, about 1 kb to about 20 kb, about 1 kb to about 15 kb, about 1kb to about 10 kb, about 1 kb to about 8 kb, about 1 kb to about 5 kb, or about 10 kb to about 15 kb.

In general, a reverse transcriptase used in the methods and compositions provided herein can be thermostable and can be capable of amplifying RNA in the presence of manganese, with high fidelity and efficiency. In some cases, a reverse transcriptase is not thermostable. A reverse transcriptase can be active up to, e.g., about 40, 45, 50, 55, 60, 65, 70, 75, 80, 85, 90, or 95°C. In some cases, a reverse transcriptase has an optimal reaction temperature of about 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90°C. In some cases, the reverse transcriptase is a hot-start reverse transcriptase. In some cases, the reverse transcriptase can have an error rate of less than one per about 10,000 to about 30,000 nucleotides and a >60% conversion rate. In some cases, the error rate of a reverse transcriptase is less than one per 60 kb, 55 kb, 50 kb, 45 kb, 40 kb, 35 kb, 30kb, 25 kb, 20 kb, 15 kb, or 10 kb. In some cases, the error rate of a reverse transcriptase is less than one per about 5 kb to about 60kb, about 10 kb to about 60 kb, about 10 kb to about 50 kb, about 10 kb to about 40 kb, about 10 kb to about 35 kb, about 10 kb to about 35 kb, about 15 kb to about 30 kb, or about 20 kb to about 30 kb. In some cases, the conversion rate is about, greater than, less than, or at least 40%, 45%, 50%, 55%, 60%, 65%, 70%, 75%, 80%, 85%, 90%, 95%, or 99%. In some cases, the conversion rate is about 40% to about 95%, about 40% to about 90%, about 40% to about 85%, about 40% to about 80%, about 40% to about 75%, about 40% to about 70%, about 40% to about 60%, about 60% to about 99%, about 60% to about 95%, about 60% to about 90%, about 60% to about 85%, about 60% to about 80%, about 60% to about 75%, or about 60% to about 70%. In some cases, the presence of magnesium in a reaction mixture can inhibit or affect the reverse transcriptase activity. In some cases, the reverse transcriptase activity is not affected, or is less affected, if all or a portion of the magnesium, although present in the reaction mixture, is inaccessible to the reverse transcriptase, as provided herein.

Some DNA-dependent DNA polymerases have RT activity; some reverse transcriptases have DNA-dependent DNA polymerase activity. Examples of such polymerases include, but are not limited to, Tth (*Thermus Thermophilus*) DNA Polymerase, Z05 DNA Polymerase (Thermus Z05), Hawk Z05 DNA Polymerase, Exonuclease Minus, or functional equivalents thereof. These enzymes can require the use of manganese divalent cation for reverse transcription, and can either use manganese for the subsequent PCR step or to bind the manganese and replace it with magnesium for the subsequent PCR step.

*T. aquaticus* (Taq) DNA polymerase, a thermostable DNA polymerase, has been reported to inefficiently synthesize cDNA using Mg²⁺ as the divalent metal ion (Jones and Foulkes, 1989, Nuc. Acids. Res. 176:8387-8388). Tse and Forget, 1990, Gene 88:293-296; and Shaffer et al., 1990, Anal. Biochem. 190:292-296, have described methods for amplifying RNA using Taq DNA polymerase and Mg²⁺ ion. However, the methods can be inefficient and insensitive.

Certain thermostable DNA polymerases can be used to efficiently reverse transcribe an RNA template by carrying out the reaction in a manganese buffer (Mn²⁺), rather than a magnesium (Mg²⁺) buffer, as can be preferred for primer extension using a DNA template.

High temperature reverse transcription methods and combined reverse transcription/amplification methods using a thermostable DNA polymerase in a Mn²⁺ buffer are known in the art (see, for example, U.S. Patent Nos. 5,310,652; 5,322,770; 5,407,800; 5,561,058; 5,641,864; and 5,693,517). There is also a disclosure of a modification of these methods, wherein the modification involves the use of mutant DNA polymerases, and the reaction is carried out in a buffer containing Mg²⁺ as the divalent cation used to activate the DNA polymerase (see EP1152062A2).

Z05 DNA polymerase can be a thermostable, thermoactive enzyme isolated from the *Thermus* species Z05 (US Pat. Nos. 5455170 and 5674738) and can be inactivated by an aptamer at temperatures lower than 55°C (Roche Product Bulletin). Z05 DNA polymerase can also behave as a reverse transcriptase in the presence of Mn²⁺. For amplification of DNA, Z05 can be used with Mg²⁺. For reactions using Z05 or other polymerase enzyme, the PCR mix or cocktail can contain either Mn²⁺ or Mg²⁺ as the divalent cation co-factor for enzyme activity. This one divalent cation strategy can be unsuccessful when used in one-step RT-PCR mix for droplet digital PCR. Detection and quantification of RNA targets by transcription and template amplification can be possible when both Mn²⁺ and Mg²⁺ were present in the mix. However, in some cases mixed cation use can require a tradeoff between a higher efficiency but lower fidelity Mn²⁺-activated reaction, and a higher fidelity Mg²⁺-activated reaction. This deficiency can be overcome by the methods and compositions provided herein.

In some cases, the enzyme used for the RT reaction according to the methods and compositions provided here in is Z05 DNA polymerase, such as Hawk ZO5 (Roche), which is a version of *Thermus* DNA Polymerase inactivated using an oligo aptamer.

### DNA-dependent DNA Polymerase

In some cases, a second enzyme used in the provided methods and compositions comprises DNA-dependent DNA polymerase activity.

A DNA polymerase can be a polymerase in a class of polymerases characterized as DNA-dependent DNA polymerases. A DNA polymerase can show a strong discrimination against using an RNA template, as can be expected from its function *in vivo.* In some cases, DNA polymerases are capable of *in vitro* reverse transcription of RNA (Karkas, 1973, Proc. Nat. Acad. Sci. USA 70:3834-3838; Gulati et al., 1974, Proc. Nat. Acad. Sci. USA 71:1035-1039; and Wittig and Wittig, 1978, Nuc. Acid Res. 5:1165-1178). For example, Gulati et al. found that *E. coli* Pol I can be used to transcribe Q beta viral RNA using oligo(dT) 10 as a primer. Wittig and Wittig have shown that *E. coli* Pol I can be used to reverse transcribe tRNA that has been enzymatically elongated with oligo(dA).

A DNA-dependent DNA polymerase used in the methods and compositions provided herein can be thermostable and can be capable of amplifying DNA in the presence of the magnesium, with high fidelity and efficiency, such as an error rate of less than one per 10,000 to 30,000 nucleotides and >60% conversion rate. In some cases, the error rate of a DNA-dependent DNA polymerase is less than one per 60 kb, 55 kb, 50 kb, 45 kb, 40 kb, 35 kb, 30kb, 25 kb, 20 kb, 15 kb, or 10 kb. In some cases, the error rate of a reverse transcriptase is less than one per about 5 kb to about 60kb, about 10 kb to about 60 kb, about 10 kb to about 50 kb, about 10 kb to about 40 kb, about 10 kb to about 35 kb, about 10 kb to about 35 kb, about 15 kb to about 30 kb, or about 20 kb to about 30 kb. In some cases, the conversion rate is about, greater than, less than, or at least 40%, 45%, 50%, 55%, 60%, 65%, 70%, 75%, 80%, 85%, 90%, 95%, or 99%. In some cases, the conversion rate is about 40% to about 95%, about 40% to about 90%, about 40% to about 85%, about 40% to about 80%, about 40% to about 75%, about 40% to about 70%, about 40% to about 60%, about 60% to about 99%, about 60% to about 95%, about 60% to about 90%, about 60% to about 85%, about 60% to about 80%, about 60% to about 75%, or about 60% to about 70%. The presence of manganese in a reaction mixture can generally inhibit or affect the reverse transcriptase activity. However, in some cases, the DNA-dependent DNA polymerase activity is not affected, or is less affected, if all or a portion of the manganese, although present in the reaction mixture, is inaccessible to the DNA polymerase, as provided herein. Suitable DNA-dependent DNA polymerases include, but are not limited to, DNA Z05, PyroPhage® 3173 DNA Polymerase, Exonuclease Minus for DNA polymerase with RT activity, and hot-start DNA polymerases such Eagle Taq, FastStart Taq or aptamer-inactivated Taq.

In some cases, the first enzyme for the RT reaction and the second enzyme for the DNA amplification (e.g. PCR reaction) are the same enzyme, such as the Hawk Z05 DNA polymerase.

Described herein are methods for reverse transcribing and amplifying an RNA, comprising: (a) contacting an RNA with: (i) an enzyme comprising both reverse transcriptase activity and DNA-dependent DNA polymerase activity, and (ii) a reaction mixture comprising manganese and magnesium, under a condition suitable for reverse transcription, thereby reverse transcribing the RNA into a DNA, wherein the magnesium is inaccessible to the enzyme during at least part of the duration of the reverse transcription reaction; and (b) amplifying the DNA with the enzyme in the reaction mixture, wherein the magnesium is accessible to the enzyme during the amplification reaction.

In certain assays, a first enzyme for an RT reaction has DNA-dependent DNA polymerase activity, but does not provide suitable activity for high-fidelity and/or high efficient PCR reaction. In those assays, a second and different enzyme can be provided.

In some cases, the second enzyme for the DNA amplification reaction (e.g., PCR) can be different from the first enzyme for the RT reaction. In some cases, the second enzyme used for the PCR step can be a hot-start DNA polymerase, such as such Eagle Taq, ITaq, FastStart Taq or aptamer-inactivated Taq. In some cases, the activity of a hot-start DNA polymerase is inhibited at ambient temperature by binding of an antibody, fragment of an antibody, or other binding partner, to the DNA polymerase, or by the presence of a covalently bound inhibitor that can dissociate after a high-temperature activation step. In some cases, heating a reaction mix (to, e.g., 95°C) denatures the antibody, antibody fragment, or binding partner. In some cases, a hot-start/cold-finish DNA polymerase is used that is inactive at ambient temperature but is activated at elongation temperature.

In some cases, two different enzymes can be used for the RT reaction and DNA amplification (e.g., PCR). The first enzyme can have high reverse transcriptase activity in the presence of manganese, but may be inhibited or become less active when in contact with magnesium. Thus, in some cases, when magnesium is contained in the reaction mix, it is inaccessible or not in contact with the first enzyme, and the magnesium does not affect the reverse transcriptase activity of the first enzyme. The second enzyme can have high DNA-dependent DNA polymerase activity in the presence of magnesium, but can be inhibited or become less active when in contact with manganese.

The amount of the enzymes (the first enzyme and/or the second enzyme) in each reaction can vary, and can be optimized on an assay-by-assay basis.

In some cases, the final concentration of the first enzyme in the reaction mixture is from about 0.0015 to about 2.0, about 0.01 to about 2.0, about 0.01 to about 1, about 0.1 to about 2.0, about 0.1 to about 1, about 0.125 to about 0.5, or about 0.5 U/µL. In some cases, the concentration of the first enzyme in the reaction mixture is about, more than, less than, or at least 0.0015, 0.01, 0.05, 0.1, 0.5, 1, 1.5, or 2 U/µL. The particular concentration can be optimized empirically for any particular reaction using routine experimental methods.

In some cases, the final concentration of second enzyme in the reaction mixture can be from 0 to about 0.04, from about 0.025 to about 0.04, or about 0.025 U/µL. In some cases, the final concentration of the second enzyme in the reaction mixture is about, more than, less than, or at least 0.001, 0.002, 0.003, 0.004, 0.005, 0.006, 0.007, 0.008, 0.009, 0.01, 0.015, 0.02, 0.025, 0.030, 0.035, 0.040, 0.045, 0.05, 0.055, 0.06, 0.065, 0.07, 0.075, 0.08, 0.085, or 0.09 U/µL The particular concentration can be optimized empirically for any particular reaction using routine experimental methods.

### Two Divalent Cation System

Provided herein is a two divalent cation system of both manganese and magnesium in a single reaction mixture of a one-step RT-PCR reaction.

Enzymes such as Tth (*Thermus Thermophilus*) DNA Polymerase, ZO5 DNA Polymerase (Thermus ZO5), or Hawk ZO5 DNA Polymerase, can require the use of manganese divalent cation for reverse transcription and can either use manganese for the subsequent PCR step or bind the manganese and replace it with magnesium for the subsequent PCR step. Magnesium can be the preferred divalent cation for the PCR step, but it can be inhibitory for the RT step. In general, the protocols known in the art do not have both metals present at the same time in a single reaction mix. For example, in some protocols, the manganese is removed after the RT reaction and/or is replaced with magnesium.

In some cases of the methods and compositions provided herein, the manganese is not removed from the reaction mixture following an RT reaction and prior to the DNA amplification reaction (e.g. PCR), such as by adding a chelator (e.g., EDTA or EGTA). In some cases, manganese is removed from a reaction mixture following an RT reaction and prior to a DNA amplification reaction.

In some cases, the reaction mixture does not contain a chelator (e.g. EDTA or EGTA). In some cases, a reaction mixture contains one or more chelators (e.g., EDTA, EGTA, or citric acid).

In a divalent cation system provided herein, magnesium can be inaccessible to a first enzyme during at least part of a duration of a reverse transcription reaction. In some cases, magnesium is inaccessible to the first enzyme during the initiation of the reverse transcription reaction, but can become accessible to the reverse transcriptase as the RT reaction progresses. In some cases, magnesium is not present in a concentration that substantially affects an RT reaction.

Described herein are formulations that can allow both manganese and magnesium divalent cations to be added to one reaction mix that can be used to perform a one-step RT-PCR reaction where the reverse transcription of the RNA to the cDNA can be performed at a first temperature, (e.g., about 55°C to about 60°C, about 50°C to about 70°C, about 50°C to about 65°C, about 50°C to about 60°C, about 60°C to about 70°C, about 55°C to about 70°C, or about 55°C to about 65°C) and the subsequent PCR can be performed using standard cycling conditions.

Without being bound by any particular theory, the reaction mixtures provided herein can have several components that bind magnesium and manganese. In some cases, the components in a reaction mix (e.g., the enzymes, BSA, dNTPs (native and analogs), and buffer (e.g. Tris)) and the order of addition of the components can create an environment where the magnesium is bound at the start of the RT reaction and the manganese is available for the RT step without the inhibition from the magnesium. As a reaction progresses, the magnesium can then be available for the subsequent DNA amplification step. Concurrently, the manganese can become less accessible to the second enzyme. Therefore, the equilibrium between the manganese and magnesium in the reaction mix can change during the RT reaction and/or amplification reaction and can provide the different cations used by the two separate enzyme activities at a proper time without being inhibitory.

In some cases, one cation preferentially binds an enzyme over another. In some cases (1) magnesium can be present in the mix and can be complexed with the one or more enzymes, BSA, dNTPs and/or Tris, and (2) the manganese can be added during the set up and can be available for the reverse transcription step. After the initial heat cycling, the manganese and magnesium can reach a different equilibrium where the manganese can be bound and the magnesium can now be available for the PCR step. Some manganese can be made unavailable after the RT step due to oxidation in a basic solution, or due to the binding of manganese to other components present in the reaction. Provided herein are unique mixes that can allow for both metals to be used in the reaction but can have separate availability during the different reaction stages.

The divalent cation can be supplied in the form of a salt such MgCl₂, Mg(OAc)₂ (magnesium acetate),MgSO₄, MgSO₄-heptahydrate, MgSO₄-6H₂O, MgBr₂, MgBr₂-6H₂0, MgCl₂-6H₂0, MgCrO₄, Mg(OH)₂, Mg(IO₃)₂, Mg₂B₂O₅, MgBr₂, MgSO₃, Mg(H₂PO₄)₂, MgHPO₄, Mg₃(PO₄)₂, MnCl₂, MgCl₂-6H₂0, MgF₂, MgI₂, Mn(OAc)₂, MnSO₄, MnBr₂, MnBr₂·4H₂O, MnCO₃, MnF₂, MnF₃ MnI₂, MnMoO₄, Mn(NO₃)₂, Mn(ClO₄)₂, MnSO₄·H₂O, (HCO₂)₂Mn, MnCl₂·4H₂O, CaCl₂, CaCO3, CaO, CoCl₂, CoF2, CoBr2, NiCl₂, NiBr2, NiCO3, NiF2, NiSO4, CuCl₂, CuBr, CuBr₂, CuF₂, CuSO₄, ZnCl₂, ZnO, ZnS, ZnF₂, Zn(NO₃)₂, Zn(ClO₃)₂, ZnSO₄, Zn₃(PO₄)₂, ZnCrO₄,CdS, CdSO₄, Cd(OAc)₂,CdCO₃, Cd(NO₃)₂, CdCl₂, SrCl₂, SrCO₃, SrF₂, or SrSO₄ In some cases, MgCl₂ and MnCl₂ are used. In some cases, Mg(OAc)₂ and Mn(OAc)₂ are used. In some cases, MgSO₄ and MnSO₄, are used. For reactions using Mn²⁺, cation concentrations in a Tris-HCl buffer can be in a range from about 0.5 to about 7 mM, about 0.1 to about 10 mM, about 1 to about 10 mM, about 1 to about 5 mM of MnCl₂, Mn(OAc)₂, or MnSO₄, or between about 0.5 and about 2 mM. In some cases, the concentration of Mn²⁺ in the reaction is about 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, or 10 mM. The concentration of Mn²⁺ in a bicine/KOAc (potassium acetate) buffer or tricine/KOAc buffer can range from about 0.5 to about 20 mM, about 0.1 to about 40 mM, about 1 to about 20 mM, about 1 to about 10 mM, about 2 to about 20 mM, about 5 to about 15 mM, about 1 to about 5 mM, or about 0.5 to about 5 mM of MnCl₂, Mn(OAc)₂, or MnSO₄.

In general, for reactions using Mg²⁺, usable divalent cation concentrations in a Tris-HCl buffer can be in a range from about 0.5 to about 10 mM, about 0.1 to about 20 mM, about 1 to about 15 mM, about 5 to about 20 mM, about 5 to about 10 mM, about 1 to about 10 mM, or about 2 to about 7 mM of MgCl₂, Mg(OAc)₂, or MgSO₄. In a bicine/KOAc or tricine/KOAC buffers, usable Mg²⁺ concentrations can be in a range from about 0.5 to about 20 mM, about 0.1 to about 20 mM, about 1 to about 15 mM, about 5 to about 20 mM, about 5 to about 10 mM, about 1 to about 10 mM, or about 2 to about 7 mM for MgCl₂, Mg(OAc)₂, or MgSO₄, or between about 0.5 and about 5 mM. In some cases, the concentration of Mg²⁺ in the reaction is about, more than, less than, or at least 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.5, 3, 3.5, 4, 4.5, 5, 5.5, 6, 6.5, 7, 7.5, 8, 8.5, 9, 9.5, or 10 mM. The optimal divalent ion concentration in a particular reaction can depend not only on the particular enzyme used, but also on the other reaction components, such as, for example, the dNTP concentration and primer sequence and concentration. One of skill will understand reaction conditions in general, and that the divalent cation concentration can vary.

In general, a reaction mix for the one-step RT-PCR can comprise a salt form of manganese, e.g., manganese acetate.

In some cases, the concentration of manganese in a reaction mixture can be from about 0.25 mM to about 0.5 mM, or about 1 mM, 2 mM, 3 mM, 4 mM, 5 mM, 6 mM, 7 mM, 8 mM, 9 mM, or 10 mM, or from about 0.4 mM to about 5mM, from about 0.5 mM to about 2 mM, from about 1 to about 5 mM, from about 1 to about 10 mM, from 1 to about 20 mM, from about 0.1 to about 5 mM, or from about 0.1 to about 1 mM.

In some cases, the final concentration of manganese in a reaction mixture is about 1 mM.

A reaction mixture can comprise one or more buffers. The one or more buffers can be, e.g., TAPS, Bicine, Tris, Tris-HCl, Tricine, TAPSO, HEPES, TES, MOPS, PIPES, Cacodylate, SSC, MES, or succinic acid. In some cases, the pKₐ of the one or more buffers is about, more than, less than, or at least 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, or 8.5. In some cases, the pKₐ of the one or more buffers is about 5 to about 9, about 6 to about 9, about 7 to about 9, or about 6.5 to about 7.5.

In general, a reaction mix for a one-step RT-PCR can comprise a salt form of magnesium, e.g., magnesium acetate.

In some cases, the concentration of magnesium in the reaction mixture can be from about 0.25 mM to about 0.5 mM, or about 1 mM, 2 mM, 3 mM, 4 mM, 5 mM, 6 mM, 7 mM, 8 mM, 9 mM, or 10 mM, or from about 0.5 mM to about 8 mM, from about 0.5 mM to about ImM, from about 1 to about 10 mM, from about 0.5 mM to about 5 mM, from about 1 to about 10 mM, from about 0.1 to about 20 mM, from about 0.5 to about 5 mM. The concentration of magnesium in the reaction mixture can be about, more than, less than, or at least 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.5, 3, 3.5, 4, 4.5, 5, 5.5, 6, 6.5, 7, 7.5, 8, 8.5, 9, 9.5, or 10 mM.

In some cases, the final concentration of magnesium in a reaction mixture is about 1 mM.

In some cases, the ratio of the molar concentration of manganese to magnesium in a reaction mixture is from about 1:2 to about 1:1, 2:1, or 3:1, from about 1:2 to about 1.5:1, from about 10:1 to about 1:10, from about 10:1 to about 1:10, from about 5:1 to about 1:10, from about 1:1 to about 1:10, from about 5:1 to about 1:5, or from about 2:1 to about 1:2. In some cases, the ratio of the concentration of manganese and magnesium in a reaction mixture is about 1:20, 1: 15, 1:12, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, 11:1, 12: 1, 15:1, 17:1, or 20:1.

In some cases, the molar ratio between the magnesium and each of the dNTPs present in the reaction mixture at the beginning of a reaction is about 1:1. In some cases, the molar ratio between the magnesium and the sum of an amount of: (1) a chelator, if present, and (2) the dNTPs (including analogs) in the reaction mixture, e.g., at the beginning of step (a), is about 1:1.

### Additional Components of a Reaction Mixture

Reaction mixtures described herein can contain additional components, e.g., one or more buffers and dNTPs, random primers, primers specific for a target sequence, as well as optional components, such as preservatives.

In some cases, a reaction mixture does not contain a chelator, such as EDTA or EGTA. In some cases, a reaction mixture comprises one or more chelators. The one or more chelators can be, e.g., EDTA, EGTA, citric acid, L-(+)-tartaric acid, potassium oxalate, sodium citrate, sodium L-tartrate, ammonium citrate, potassium tetraoxalate, ethylene glycol-bis(2-aminoethylether)-*N,N,N',N'*-tetraacetic acid, potassium citrate, potassium oxalate, sodium bitartrate, 5-sulfosalicylic acid, or nitrilotriacetic acid.

A reaction mixture can comprise one or more dNTPs. dNPTs can include dATP, dCTP, dGTP, dTTP, as well as their analogs. The concentration of each dNTP in a reaction mixture can be from about 0.05 mM to about 0.4 mM, from about 0.1 mM to about 0.2 mM, from about 0.1 mM to about 0.5 mM, or from 0.05 mM to about 0.5 mM. In some cases, the concentration of dNTPs in a reaction is about, more than, less than, or at least 0.01 mM, 0.025 mM, 0.05 mM, 0.075 mM, 0.1 mM, 0.125 mM, 0.15 mM, 0.175 mM, 0.2 mM, 0.225 mM, 0.25 mM, 0.275 mM, 0.3 mM, 0.325 mM, 0.35 mM, 0.375 mM, 0.4 mM, 0.425 mM, 0.45 mM, 0.475 mM, or 0.5 mM. In some cases, a reaction mixture comprises dUTP. Addition of dUTP to a reaction mixture can provide a means for eliminating carry-over amplicon or nucleic acid contamination generated from a previous reaction. In some cases, uracil-N-glycocylase can be used to degrade a uracil-containing nucleic acid carried over from previous reactions without affecting the native nucleic acid templates. The final concentration of dUTP in a reaction mixture can be from about 0.05 mM to about 0.4 mM, from about 0.1 mM to about 0.2 mM, from about 0.1 mM to about 0.5 mM, or from 0.05 mM to about 0.5 mM. The final concentration of dNTP can be about, more than, less than, or at least 0.01 mM, 0.025 mM, 0.05 mM, 0.075 mM, 0.1 mM, 0.125 mM, 0.15 mM, 0.175 mM, 0.2 mM, 0.225 mM, 0.25 mM, 0.275 mM, 0.3 mM, 0.325 mM, 0.35 mM, 0.375 mM, 0.4 mM, 0.425 mM, 0.45 mM, 0.475 mM, or 0.5 mM.

In some cases, a reaction mixture comprises one or more dNTP analogs in addition to native dNTPs. The one or more dNTP analogs can be, e.g.,7-deaza-dGTP to facilitate the amplification of GC rich target sequences. The final concentration of 7-deaza-dGTP in the reaction mixture can be, e.g., from about 0.05 mM to about 0.4 mM or from about 0.1 mM to about 0.2 mM.

The amount of one or more dNTPs in each reaction can vary, and the amount can be optimized on an assay-by-assay basis. In general, the final concentration of each dNTP in the reaction mixture can be, e.g., from about 0.1 mM to about 0.5 mM or from about 0.2 mM to about 0.4 mM.

In some cases, a reaction mixture comprises a Tricine buffer, with a suitable pH from about 7 to about 9, or about pH 8.3. In some cases, a Tricine based buffer is not used.

In some cases, a reaction mixture comprises a Tris based buffer, e.g., Tris-HCl, with a suitable pH from about 7 to about 9, or about pH 8.3. In some cases, a Tris based buffer is not used.

The amount the monovalent cation (e.g., K+ or Na+) in each reaction can vary, and the amount can be optimized on an assay-by-assay basis. In general, the final concentration of monovalent cation (e.g., K+ or Na+) in a reaction mixture can be, e.g., from about 5 mM to about 500 mM or from about 50 mM to about 200 mM.

The pH of a reaction mix can from about 5 to about 10, from about 6 to about 9, from about 7 to about 8, or from about 7 to about 10. The pH of the reaction mix can be about, more than, less than, or at least 5, 5.5, 6, 6.1, 6.2, 6.3, 6.4, 6.5, 6.6, 6.7, 6.8, 6.9, 7, 7.1, 7.2, 7.3, 7.4, 7.5, 7.6, 7.8, 7.9, 8, 8.1, 8.2, 8.3, 8.4, 8.5, 8.6, 8.7, 8.8, 8.9, 9, 9.5, 10, 10.5, or 11.

In some cases, a reaction mixture comprises glycerol. The amount of glycerol in a reaction can vary, and the amount can be optimized on an assay-by-assay basis. In general, a final concentration of glycerol in a reaction mixture can be about 2% to about 20%, about 5% to about 10%, or about 5% to about 15%. In some cases, the concentration of glycerol is about, more than, less than, or at least 1, 2, 3, 4, 5, 6, 7, 8, 9, 10, 11, 12, 13, 14, 15, 16, 17, 18, 19, or 20%

In some cases, a reaction mixture comprises BSA. The amount of BSA in a reaction can vary, and the amount can be optimized on an assay-by-assay basis. In general, the final concentration of BSA in the reaction mixture can be from about 0.1 to about 5.0 mg/mL, from about 0.2 to about 1.0 mg/mL, about 1.0 to about 2.0 mg/mL, about 2.0 to about 3.0 mg/mL, about 3.0 to about 4.0 mg/mL, about 0.3 to about 2.0 mg/mL, or about 0.5 to about 3.0 mg/mL. In some cases, the concentration of BSA in the mix is about, more than, less than, or at least 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.5, or 5 mg/mL.

In some cases, a reaction mixture comprises a detergent. The amount of the detergent in a reaction can vary, and the amount can be optimized on an assay-by-assay basis. In general, the final concentration of detergent in a reaction mixture can be, e.g., from about 0.01% to about 2% or from about 0.1% to about 0.75%. The detergent can be a non-ionic detergent. The detergent can be, e.g., Tween-20, NP-40, or Triton X-100, Brij 52, Brij 58, or Brij 98.

In some cases, a reaction mix can contain random hexamers and/or poly T primers. In general, the final concentration of hexamers in a reaction mixture can be from about 10 ng to about 250 ng, about 50 ng to about 100 ng per reaction, about 1 to about 50 ng/reaction, about 50 to about 250 ng/reaction, about 100 to about 250 ng/reaction, about 150 to about 250 ng/reaction, about 175 to about 250 ng/reaction, about 25 to about 100 ng/reaction.

In some cases, one or more target-specific primers (e.g., one or more of the same primers used for amplification) can be used to initiate reverse transcription. The final concentration of each of the one or more primers in the reaction can range from about 250 nM to about 1250 nM, about 500 nM to about 1000 nM, about 100 nM to about 2000 nM, about 100 nM to about 1000 nM, about 500 nM to about 2000 nM, about 1500 to about 2000 nM, or about 750 to about 1000 nM. In some cases, the final concentration of the one or more primers in the reaction can be about, more than, less than, or at least 100, 200, 300, 400, 500, 600, 700, 800, 900, 1000, 1100, 1200, 1300, 1400, 1500, 1600, 1700, 1800, 1900, or 2000 nM.

In some cases, a reaction mixture can contain one or more RNase inhibitors, e.g., to protect RNA samples from nuclease activity. An RNase inhibitor can be a specific protein that can tightly bind a ribonuclease, rendering it inactive. In general, the final concentration of RNase inhibitor in a reaction mixture can be from about 0.05 U/uL to about 2.0 U/uL, or from about 0.2 U/µL to about 0.5 U/µL. In some cases, the final concentration of the one or more RNase inhibitors in a reaction mix is about, more than, less than, or at least 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3., 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, or 5 U/µL.

Additional components that can be added to a reaction mixture are provided below.

In general, an RT-PCR reaction mix can be prepared in two steps. In the first step, an RT-PCR master mix that lacks manganese, and a separate manganese salt solution (e.g., manganese acetate), can be prepared separately. The manganese salt solution can be added to the RT-PCR mix before the assay is performed. Without being bound by any particular theory, it is believed that manganese is not stable in the master mix due to a basic pH. Thus, manganese can be added shortly prior to the assay being carried out. In some cases, the pH of the master mix is about, more than, less than, or at least 13, 12.5, 12, 11.5, 11, 10.5, 10, 9.5, 9, 8.5, 8, 7.5, 7, 6.9, 6.8, 6.7, 6.6, 6.5, 6.4, 6.3, 6.2, 6.1, 6, 5.9, 5.8, 5.7, 5.6, 5.5, 5.4, 5.3, 5.2, 5.1, 5, 4.9, 4.8, 4.7, 4.6, 4.5, 4.4, 4.3, 4.2, 4.1, 4, 3.9, 3.8, 3.7, 3.6, 3.5, 3.4, 3.3, 3.2, 3.1, or 3.

In some cases, shortly before carrying out an RT-PCR assay, such as prior to generation of droplets in a ddPCR, the two solutions (the master mix without manganese and the manganese salt solution) are mixed to generate the master mix. The amount of time between the addition of manganese salt solution to the master mix without manganese and generation of droplets or the carrying out the RT-PCR reactions can be as short as possible, such as about, less than, more than, or at least 1, 2, 5, 10, 15, 20, 25, 30, 40, or 50 seconds, or about, less than, more than, or at least 1, 2, 3, 5, 8, 10, 15, 20, 25, 30, 40, 50, or 60 minutes. In some cases, the amount of time between the addition of manganese salt solution to the master mix without manganese and generation of droplets is about 1 sec to about 60 sec, about 1 sec to about 50 sec, about 1 sec to about 40 sec, about 1 sec to about 30 sec, about 1 sec to about 20 sec, about 1 sec to about 10 sec, about 10 sec to about 60 sec, about 20 sec to about 60 sec, about 30 sec to about 60 sec, about 40 sec to about 60 sec, or about 50 sec to about 60.

One or more pairs of primers specific to a target sequence to be amplified can also be included in the PCR reactions. In some cases, a plurality of pairs of primers is added, e.g., about, more than, less than, or at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200, 300, 400, 500, 600, 700, 800, 900, 1000, 2000, 3000, 4000, 5000, 6000, 7000, 8000, 9000, or 10,000. The proper amount of primers added can be optimized empirically for any particular reaction using routine experimental methods.

The amount of RNA in a reaction can be from about 1 fg to about 10 µg, about 1 pg to about 1 µg, about 10 fg to about 100 ng, or about 1 fg to about 10 µg. The amount of RNA in a reaction can be about, more than, less than, or at least 1 fg, 5 pg, 10 fg, 50 fg, 100 fg, 500 fg, 1 pg, 5 pg, 10 pg, 50 pg, 100 pg, 500 pg, 1 ng, 5 ng, 10 ng, 50 ng, 100 ng, 500 ng, 1 µg, 5 µg, or 10 µg.

### Sample Preparation

Samples to be analyzed using the methods, compositions, and kits described herein can be derived from a non-cellular entity comprising nucleic acid (e.g., a virus) or from a cell-based organism (e.g., member of archaea, bacteria, or eukarya domains). The sample can be from a plant, fungi, eubacteria, archeabacteria, protist, or animal. The animal can be a fish, e.g., a zebrafish. The animal can be a mammal. The mammal can be, e.g., a dog, cat, horse, cow, mouse, rat, or pig. The mammal can be a primate, e.g., a human, chimpanzee, orangutan, or gorilla. The human can be a male or female. The sample can be from a human embryo or human fetus. The human can be an infant, child, teenager, adult, or elderly person. A sample can be obtained from an animal that is pregnant. The pregnant animal can be a human. The pregnant human can be at about week 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 of gestation.

The sample can be aqueous humour, vitreous humour, bile, whole blood, blood serum, blood plasma, serum, breast milk, cerebrospinal fluid, cerumen, enolymph, perilymph, gastric juice, mucus, peritoneal fluid, saliva, sebum, semen, sweat, tears, vaginal secretion, vomit, feces, or urine.

The sample can be obtained from a hospital, laboratory, clinical or medical laboratory. In some cases, the sample can be taken from a subject.

The sample can comprise nucleic acid, e.g., RNA and/or DNA. The sample can comprise cell-free nucleic acid, e.g., cell-free RNA and/or cell-free DNA. In some cases, the sample is obtained from a swab of a surface, such as a door or bench top. The sample can be a cell line, total RNA, cell-free plasma RNA, FFPE sample, or flash frozen sample. The sample can be from an organ, e.g., heart, skin, liver, lung, breast, stomach, pancreas, bladder, colon, gall bladder, brain, etc.

The methods described herein can be used for analyzing one or more target nucleic acid molecules, e.g., RNA molecules, including, but not limited to mRNA, tRNA, rRNA, non-coding RNA such as siRNA, micro RNA, piwi-interacting RNA (piRNA), small nuclear ribonucleic acid (sn RNA), small hairpin RNA (short hairpin RNA; shRNA), subgenomic mRNA, spliced RNA, unspliced RNA, exosomal RNA, as well viral genome RNA. The RNA can be cell-free RNA derived from a blood sample. RNA can include spliced RNA and unspliced RNA. In some cases, the RNA is synthetic.

In general, the RNA to be amplified can contain one or more target sequences of interest. The length of the sequence can vary, depending on the particular application of the RT-PCR reaction. In some cases, the length of the target sequence is about, more than, less than, or at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200, 300, 400, 500, 600, 700, 800, 900, 1000, 2000, 3000, 4000, 5000, 6000, 7000, 8000, 9000, or 10,000 bases in length.

In some cases, the sample is a biopsy. A biopsy can be preserved by formalin-fixing and paraffin-embedding (FFPE) a tissue obtained in a biopsy. A biopsy can be processed into smaller pieces. In some cases, the biopsy can be treated to preserve RNA, e.g., with RNAlater® or RNASafer™. In some cases, the sample comprises one or more reagents that inhibit RNases. The biopsy can be stored on wet ice (approximately 4°C), at room temperature (approximately 25°C), at approximately -20°C, at approximately -80°C, e.g., stored on dry ice, in liquid nitrogen or a dry ice/alcohol slurry.

If the tissue is frozen, the tissue can be frozen within about 0.5, 1, 5, 10, 15, 30, 60, 120, 150, 180, 210, or 240 minutes of surgical resection. Fixative agents that can be used on the biopsy tissue include, e.g., methanol-acetone, ethanol, acetone, formalin, Carnoy's fixative (60% ethanol, 30% chloroform, 10% glacial acetic acid), Bouin's fixative, methacarn (substitute 60% methanol for the ethanol in Carnoy), FINEfix, Omnifix, and UMFIX (universal molecular fixative).

The methods and compositions described herein can be used to evaluate a quantity of polynucleotides (e.g., RNA, mRNA, siRNA, miRNA, cRNA, single-stranded DNA, single-stranded RNA, double-stranded RNA, tRNA, rRNA, cDNA, etc.). The methods and compositions can be used to evaluate a quantity of a first polynucleotide compared to the quantity of a second polynucleotide. The methods can be used to analyze the quantity of synthetic plasmids in a solution; to detect a pathogenic organism (e.g., microbe, bacteria, virus, parasite, retrovirus, lentivirus, HIV-1, HIV-2, influenza virus, etc.) within a sample obtained from a subject or obtained from an environment. The methods also can be used in other applications wherein a rare population of polynucleotides exists within a larger population of polynucleotides.

The RNA sample can be prepared using methods known in the art.

For example, plasma RNA extraction is described in Enders et al. (2003), Clinical Chemistry 49:727-731. Briefly, plasma harvested after centrifugation steps can be mixed with Trizol LS reagent (Invitrogen) and chloroform. The mixture can be centrifuged, and the aqueous layer transferred to new tubes. Ethanol is added to the aqueous layer. The mixture can then be applied to an RNeasy mini column (Qiagen) and processed according to the manufacturer's recommendations.

The methods described herein can be used for analyzing or detecting one or more target nucleic acid molecules. The term polynucleotide, or grammatical equivalents, can refer to at least two nucleotides covalently linked together. A nucleic acid described herein can contain phosphodiester bonds, although in some cases, as outlined below (for example in the construction of primers and probes such as label probes), nucleic acid analogs are included that can have alternate backbones, comprising, for example, phosphoramide (Beaucage et al., Tetrahedron 49(10):1925 (1993) and references therein; Letsinger, J. Org. Chem. 35:3800 (1970); Sprinzl et al., Eur. J. Biochem. 81:579 (1977); Letsinger et al., Nucl. Acids Res. 14:3487 (1986); Sawai et al, Chem. Lett. 805 (1984), Letsinger et al., J. Am. Chem. Soc. 110:4470 (1988); and Pauwels et al., Chemica Scripta 26:141 91986)), phosphorothioate (Mag et al., Nucleic Acids Res. 19:1437 (1991); and U.S. Pat. No. 5,644,048), phosphorodithioate (Briu et al., J. Am. Chem. Soc. 111:2321 (1989), O-methylphophoroamidite linkages (see Eckstein, Oligonucleotides and Analogues: A Practical Approach, Oxford University Press), and peptide nucleic acid (also referred to herein as "PNA") backbones and linkages (see Egholm, J. Am. Chem. Soc. 114:1895 (1992); Meier et al., Chem. Int. Ed. Engl. 31:1008 (1992); Nielsen, Nature, 365:566 (1993); Carlsson et al., Nature 380:207 (1996), all of which are incorporated by reference). Other analog nucleic acids include those with bicyclic structures including locked nucleic acids (also referred to herein as "LNA"), Koshkin et al., J. Am. Chem. Soc. 120.13252 3 (1998); positive backbones (Denpcy et al., Proc. Natl. Acad. Sci. USA 92:6097 (1995); non-ionic backbones (U.S. Pat. Nos. 5,386,023, 5,637,684, 5,602,240, 5,216,141 and 4,469,863; Kiedrowshi et al., Angew. Chem. Intl. Ed. English 30:423 (1991); Letsinger et al., J. Am. Chem. Soc. 110:4470 (1988); Letsinger et al., Nucleoside & Nucleotide 13:1597 (1994); Chapters 2 and 3, ASC Symposium Series 580, "Carbohydrate Modifications in Antisense Research", Ed. Y. S. Sanghui and P. Dan Cook; Mesmaeker et al., Bioorganic & Medicinal Chem. Lett. 4:395 (1994); Jeffs et al., J. Biomolecular NMR 34:17 (1994); Tetrahedron Lett. 37:743 (1996)) and non-ribose backbones, including those described in U.S. Pat. Nos. 5.235,033 and 5,034,506, and Chapters 6 and 7, ASC Symposium Series 580, "Carbohydrate Modifications in Antisense Research", Ed. Y. S. Sanghui and P. Dan Cook. Nucleic acids containing one or more carbocyclic sugars are also included within the definition of nucleic acids (see Jenkins et al., Chem. Soc. Rev. (1995) pp 169 176). Several nucleic acid analogs are described in Rawls, C & E News Jun. 2, 1997 page 35. "Locked nucleic acids" are also included within the definition of nucleic acid analogs. LNAs are a class of nucleic acid analogues in which the ribose ring is "locked" by a methylene bridge connecting the 2'-0 atom with the 4'-C atom. All of these references are hereby expressly incorporated by reference. These modifications of the ribose-phosphate backbone can be done to increase the stability and half-life of such molecules in physiological environments. For example, PNA:DNA and LNA-DNA hybrids can exhibit higher stability and thus can be used in some cases. The target nucleic acids can be single stranded or double stranded, as specified, or contain portions of both double stranded or single stranded sequence. Depending on the application, the nucleic acids can be DNA (including, e.g., genomic DNA, mitochondrial DNA, and cDNA), RNA (including, e.g., mRNA and rRNA) or a hybrid, where the nucleic acid contains any combination of deoxyribo- and ribo-nucleotides, and any combination of bases, including uracil, adenine, thymine, cytosine, guanine, inosine, xathanine hypoxathanine, isocytosine, isoguanine, etc.

### RT Reaction

The temperature to carry out an RT reaction can depend on the reverse transcriptase being used. In some cases, a thermostable reverse transcriptase is used and the RT reaction is carried out at about 40 °C to about 80 °C, about 45 °C to about 80 °C, about 50 °C to about 75 °C, about 55 °C to about 75 °C, about 60 °C to about 75 °C, about 60 °C to about 70 °C, about 55 °C to about 60 °C, or about 60°C. In some cases, an RT reaction is carried out at about, more than, less than, or at least 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90°C. In some cases, an RT reaction is carried out at about 35°C to about 65°C, about 35°C to about 50 °C, about 35°C to about 45°C, about 40 and 52 °C, about 45°C to about 50°C, or about 35°C to about 40°C.

An RT reaction and a PCR reaction can be carried out in various formats known in the art, such as in a partition, tube, microtiter plate, well of a microtiter plate, microfluidic device, reaction chamber in a microfluidic device, or droplets.

As described herein, an RT reaction can comprise template RNA, one or more primers, and a dNTP nix. The RT reaction can comprise nuclease-free water. In some cases, the RT reaction comprises an RNase inhibitor.

An RT reaction may be carried out in standard recommended volumes ranging from about 5 µL to about 100 µL, or in about 10 µL to about 20 µL reaction volumes. In droplets, reaction volumes can range from about 1 pL to about100 nL, or about 10 pL to about 1 nL. In some cases, an RT reaction is carried out in a droplet having a volume that is about or less than 1 nL. In some cases, the reaction volume is about, more than, less than, or at least 1, 2, 3, 4, 5, 10, 15, 20, 25, 30, 35, 40, 45, 50, 55, 60, 65, 70, 75, 80, 85, 90, 95, 100, 105, 110, 115, 120, 125, 130, 140, 150, 160, 170, 180, 190, or 200 µL. In some cases, the reaction volume is about, more than, less than, or at least 5, 10, 15, 20, 25, 30, 35, 40, 45, 50, 55, 60, 65, 70, 75, 80, 85, 90, 95, 100, 105, 110, 115, 120, 125, 130, 140, 150, 160, 170, 180, 190, 200, 300, 400, 500, 600, 700, 800, 900, 1000, 2000, 3000, 4000, 5000, 6000, 7000, 8000, 9000, or 10,000 pL.

In some cases, a PCR reaction is in a droplet having a reaction volume ranging from about 1 pL to about 100 nL, or about 10 pL to about 1 nL. In some cases, a PCR reaction is carried out in a droplet having a volume that is about or less than 1 nL. In some cases, a PCR reaction is carried out in a droplet having a volume that is about, more than, less than, or at least 1, 2, 3, 4, 5, 10, 15, 20, 25, 30, 35, 40, 45, 50, 55, 60, 65, 70, 75, 80, 85, 90, 95, 100, 105, 110, 115, 120, 125, 130, 140, 150, 160, 170, 180, 190, 200, 300, 400, 500, 600, 700, 800, 900, 1000, 2000, 3000, 4000, 5000, 6000, 7000, 8000, 9000, or 10,000 pL, 20,000, 30,000, 40,000, 50,000, 60,000, 70,000, 80,000, 90,000, or 100,000 pL.

In some cases, an RT reaction and a PCR reaction are carried out in the same partition (e.g., droplet) having a reaction volume ranges from about 1 pL to about 100 nL or about 10 pL to about 1 nL. In some cases, an RT reaction and a PCR reaction are carried out in a partition (e.g., droplet) having a volume that is about or less than 1 nL, or a volume that is about or less than 1 nL. In some cases, an RT reaction and a PCR reaction are carried out in the same partition (e.g., droplet) having a volume that is about, more than, less than, or at least 1, 2, 3, 4, 5, 10, 15, 20, 25, 30, 35, 40, 45, 50, 55, 60, 65, 70, 75, 80, 85, 90, 95, 100, 105, 110, 115, 120, 125, 130, 140, 150, 160, 170, 180, 190, 200, 300, 400, 500, 600, 700, 800, 900, 1000, 2000, 3000, 4000, 5000, 6000, 7000, 8000, 9000, 10,000, 20,000, 30,000, 40,000, 50,000, 60,000, 70,000, 80,000, 90,000, or 100,000 pL.

In some cases, an RT reaction and a PCR reaction are carried out in a plurality of droplets each having a reaction volume that ranges from about 1 pL to about 100 nL or about10 pL to about 1 nL. In some cases, an RT reaction and a PCR reaction are carried out in a plurality of droplets each having a volume that is about or less than 1 nL. In some cases, an RT reaction and a PCR reaction are carried out in a plurality of droplets each having a volume that is about, more than, less than, or at least 1, 2, 3, 4, 5, 10, 15, 20, 25, 30, 35, 40, 45, 50, 55, 60, 65, 70, 75, 80, 85, 90, 95, 100, 105, 110, 115, 120, 125, 130, 140, 150, 160, 170, 180, 190, 200, 300, 400, 500, 600, 700, 800, 900, 1000, 2000, 3000, 4000, 5000, 6000, 7000, 8000, 9000, 10,000. 20,000, 30,000, 40,000, 50,000, 60,000, 70,000, 80,000, 90,000, or 100,000 pL. In some cases, an RT reaction is heated, for example to 95 °C for 5 to 10 minutes, to inactivate the reverse transcriptase before a PCR reaction is performed.

### PCR Reaction/Amplification

The cDNA generated by RT can be amplified, e.g., by using PCR.

Techniques for amplification of sequences, including target and reference sequences, are known in the art and include the methods described, e.g., in U.S. Patent No. 7,041,481. Briefly, the techniques include methods and compositions that can separate samples into small droplets, in some instances with each containing on average less than one nucleic acid molecule (polynucleotide) per droplet, amplifying the nucleic acid sequence in each droplet, and detecting the presence of a target nucleic acid sequence. In some instances, each partition (e.g., droplet) contains on average less than one target nucleic acid molecule per partition (e.g., droplet). In some cases, each partition (e.g., droplet) contains, on average, 0 or about 0.5, 1, 1.5, 2, 2.5, 3, 3.5, 4, 4.5, 5, 5.5, 6, 6.5, 7, 7.5, 8, 8.5, 9, 9.5, or 10 target nucleic acid molecules per partition (e.g., droplet). In some cases, each partition (e.g., droplet) contains on average 0 or about 0.5, 1, 1.5, 2, 2.5, 3, 3.5, 4, 4.5, 5, 5.5, 6, 6.5, 7, 7.5, 8, 8.5, 9, 9.5, or 10 nucleic acid molecules.

Primers can be designed according to known parameters for avoiding secondary structures and self-hybridization. Different primer pairs can anneal and melt at about the same temperatures, for example, within about 1, 2, 3, 4, 5, 6, 7, 8, 9 or 10°C of another primer pair. In some cases, about, more than, less than, or at least 1, 2, 3, 4, 5, 6, 7, 8, 9, 10, 15, 20, 25, 30, 35, 40, 45, 50, 100, 200, 500, 1000, 5000, or 10,000 primers are initially used. Such primers can be used to hybridize to the genetic targets described herein.

Primers can be prepared by a variety of methods including but not limited to cloning of appropriate sequences and direct chemical synthesis using methods well known in the art (Narang et al., Methods Enzymol. 68:90 (1979); Brown et al., Methods Enzymol. 68:109 (1979)). Primers can also be obtained from commercial sources. The primers can have an identical melting temperature. The lengths of the primers can be extended or shortened at the 5' end or the 3' end to produce primers with desired melting temperatures. One of the primers of a prime pair can be longer than the other primer. In some cases, the 5' end of a primer does not anneal to a target sequence. The 3' annealing lengths of the primers, within a primer pair, can differ. Also, the annealing position of each primer pair can be designed such that the sequence and length of the primer pairs yield the desired melting temperature. An equation for determining the melting temperature of primers smaller than 25 base pairs is the Wallace Rule (Td=2(A+T)+4(G+C)). Computer programs can also be used to design primers, including but not limited to Array Designer Software (Arrayit Inc.), Oligonucleotide Probe Sequence Design Software for Genetic Analysis (Olympus Optical Co.), NetPrimer, and DNAsis from Hitachi Software Engineering. The TM (melting or annealing temperature) of each primer can be calculated using software programs such as Net Primer (free web based program at http://www.premierbiosoft.com/netprimer/index.html). The annealing temperature of the primers can be recalculated and increased after any cycle of amplification, including but not limited to cycle 1, 2, 3, 4, 5, cycles 6-10, cycles 10-15, cycles 15-20, cycles 20-25, cycles 25-30, cycles 30-35, or cycles 35-40. After the initial cycles of amplification, the 5' half of the primers can be incorporated into the products from each loci of interest, thus the TM can be recalculated based on both the sequences of the 5' half and the 3' half of each primer. Primer annealing temperatures can be about, more than, less than, or at least 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70°C. Annealing times can be about 10, 20, 30, 40, 50, 60, 70, 80, 90, 100, 110, 120, 130, 140, 150, or 160 sec. Extension temperatures can be about, more than, less than, or at least 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80°C. Extension temperatures can be about 55 to about 60 °C. Extension times can be about, more than, less than, or at least 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 550, or 600 sec.

The annealing temperature of the primers can be recalculated and increased after any cycle of amplification, including but not limited to cycle 1, 2, 3, 4, 5, cycles 6-10, cycles 10-15, cycles 15-20, cycles 20-25, cycles 25-30, cycles 30-35, or cycles 35-40. After the initial cycles of amplification, the 5' half of the primers can be incorporated into the products from each loci of interest; thus the TM can be recalculated based on both the sequences of the 5' half and the 3' half of each primer. Any DNA polymerase that catalyzes primer extension can be used including but not limited to E. coli DNA polymerase, Klenow fragment of E. coli DNA polymerase 1, T7 DNA polymerase, T4 DNA polymerase, Taq polymerase, Pfu DNA polymerase, Vent DNA polymerase, bacteriophage 29, REDTaq™. Genomic DNA polymerase, or sequenase. In some cases, a thermostable DNA polymerase is used. A hot start PCR can also be performed wherein the reaction is heated to 95°C. for two minutes prior to addition of the polymerase or the polymerase can be kept inactive until the first heating step in cycle 1. Hot start PCR can be used to minimize nonspecific amplification. Any number of PCR cycles can be used to amplify the DNA, e.g., about, more than, or less than, or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44 or 45 cycles. The number of amplification cycles can be about 1 to about 45, about 10 to about 45, about 20 to about 45, about 30 to about 45, about 35 to about 45, about 10 to about 40, about 10 to about 30, about 10 to about 25, about 10 to about 20, about 10 to about 15, about 20 to about 35, about 25 to about 35, about 30 to about 35, or about 35 to about 40.

Amplification of target nucleic acids can be performed by any means known in the art. Target nucleic acids can be amplified by polymerase chain reaction (PCR) or isothermal DNA amplification. Examples of PCR techniques that can be used include, but are not limited to, quantitative PCR, quantitative fluorescent PCR (QF-PCR), multiplex fluorescent PCR (MF-PCR), real time PCR, single cell PCR, restriction fragment length polymorphism PCR (PCR-RFLP), PCR-RFLP/RT-PCR-RFLP, hot start PCR, nested PCR, in situ polony PCR, in situ rolling circle amplification (RCA), bridge PCR, picotiter PCR, digital PCR, droplet digital PCR, and emulsion PCR. Other suitable amplification methods include the ligase chain reaction (LCR), transcription amplification, molecular inversion probe (MIP) PCR, self-sustained sequence replication, selective amplification of target polynucleotide sequences, consensus sequence primed polymerase chain reaction (CP-PCR), arbitrarily primed polymerase chain reaction (AP-PCR), degenerate oligonucleotide-primed PCR (DOP-PCR) and nucleic acid based sequence amplification (NABSA). Other amplification methods that can be used herein include those described in U.S. Pat. Nos. 5,242,794; 5,494,810; 4,988,617; and 6,582,938. Amplification of target nucleic acids can occur on a bead. In other cases, amplification does not occur on a bead. Amplification may be isothermal amplification, e.g., isothermal linear amplification.

Thermocycling reactions can be performed on samples contained in droplets. The droplets can remain intact during thermocycling. Droplets can remain intact during thermocycling at densities of greater than about 10,000 droplets/mL, 100,000 droplets/mL, 200,000 droplets/mL, 300,000 droplets/mL, 400,000 droplets/mL, 500,000 droplets/mL, 600,000 droplets/mL, 700,000 droplets/mL, 800,000 droplets/mL, 900,000 droplets/mL, 1,000,000 droplets/mL, 5 million droplets/mL, 10 million droplets/mL, 25 million droplets/mL, 50 million droplets/mL, 100 million droplets/mL, 250 million droplets/mL, 500 million droplets/mL, 750 million droplets/mL, 1 billion droplets/mL, 2.5 billion droplets/mL, 5 billion droplets/mL, 7.5 billion droplets/mL, or 10 billion droplets/mL. In some cases, droplet density is about 10,000 droplets/mL to about 100,000 droplets/mL, about 100,000 droplets/mL to about 1 million droplets/mL, about 1 million droplets/mL to about 10 million droplets/mL, about 10 million droplets/mL to about 100 million droplets/mL, about 100 million droplets/mL to about 1 billion droplets/mL, or about 1 billion droplets/mL to about 10 billion droplets/mL. In other cases, two or more droplets can coalesce during thermocycling. In other cases, greater than 100 or greater than 1,000 droplets can coalesce during thermocycling.

Universal probes can be designed by methods known in the art. In some cases, the probe is a random sequence. The universal probe can be selected to ensure that it does not bind the target polynucleotide in an assay, or to other non-target polynucleotides likely to be in a sample. A label (Fluorophore, dye) used on a probe (e.g., a Taqman probe) to detect a target nucleic acid sequence or reference nucleic acid sequence in the methods described herein can be, e.g., 6-carboxyfluorescein (FAM), tetrachlorofluorescin (TET), 4,7,2'-trichloro-7'-phenyl-6-carboxyfluorescein (VIC), HEX, Cy3, Cy 3.5, Cy 5, Cy 5.5, Cy 7, tetramethylrhodamine, ROX, and JOE. The label can be an Alexa Fluor dye, e.g., Alexa Fluor 350, 405, 430, 488, 532, 546, 555, 568, 594, 633, 647, 660, 680, 700, and 750. The label can be Cascade Blue, Marina Blue, Oregon Green 500, Oregon Green 514, Oregon Green 488, Oregon Green 488-X, Pacific Blue, Rhodamine Green, Rhodol Green, Rhodamine Green-X, Rhodamine Red-X, and Texas Red-X. A probe can be a hydrolysis probe, e.g., a 5' hydrolysis probe; a Taqman probe can be a 5' hydrolysis probe.

Fluorescence detection can be achieved using a variety of detector devices equipped with a module to generate excitation light that can be absorbed by a fluorescer, as well as a module to detect light emitted by the fluorescer. In some cases, samples (such as droplets) can be detected in bulk. For example, samples can be allocated in plastic tubes that are placed in a detector that measures bulk fluorescence from plastic tubes. In some cases, one or more samples (such as droplets) can be partitioned into one or more wells of a plate, such as a 96-well or 384-well plate, and fluorescence of individual wells can be detected using a fluorescence plate reader.

In some cases, the detector further comprises handling capabilities for droplet samples, with individual droplets entering the detector, undergoing detection, and then exiting the detector. For example, a flow cytometry device can be adapted for use in detecting fluorescence from droplet samples. In some cases, a microfluidic device equipped with pumps to control droplet movement is used to detect fluorescence from droplets in single file. In some cases, droplets are arrayed on a two-dimensional surface and a detector moves relative to the surface, detecting fluorescence at each position containing a single droplet.

Following acquisition of fluorescence detection data, a computer can be used to store and process the data. A computer-executable logic can be employed to perform such functions as subtraction of background fluorescence, assignment of target and/or reference sequences, and quantification of the data. A computer can be useful for displaying, storing, retrieving, or calculating diagnostic results from the molecular profiling; displaying, storing, retrieving, or calculating raw data from genomic or nucleic acid expression analysis; or displaying, storing, retrieving, or calculating any sample or patient information useful in the methods provided herein.

Following digital PCR of samples having primers to amplify a target nucleic acid sequence and a reference nucleic acid sequence, the number of positive samples having a target nucleic acid sequence and the number of positive samples having a reference nucleic acid sequence can be compared.

Methods of determining copy number differences by amplification are described, e.g., in U.S. Patent Application Publication No. 20100203538. Methods for determining copy number variation are described in U.S. Patent No. 6,180,349; Taylor et al. (2008) PLoS One 3(9): e3179.

In some cases, the methods provided herein permit determination of number of copies of an RNA molecule without a standard curve.

### Droplet Generation

In some cases, both an RT reaction and a DNA amplification reaction (e.g., PCR) are carried out in droplets, such as in droplet digital PCR. The droplets used herein can include emulsion compositions (or mixtures of two or more immiscible fluids) as described, e.g., in U.S. Patent No. 7,622,280. The droplets can be generated by devices described in WO/2010/036352. The term emulsion, as used herein, can refer to a mixture of immiscible liquids (such as oil and water). Oil-phase and/or water-in-oil emulsions can allow for the compartmentalization of reaction mixtures within aqueous droplets. The emulsions can comprise aqueous droplets within a continuous oil phase. The emulsions provided herein can be oil-in-water emulsions, wherein the droplets are oil droplets within a continuous aqueous phase. The droplets provided herein can be designed to prevent mixing between compartments, with each compartment protecting its contents from evaporation and coalescing with the contents of other compartments.

The mixtures or emulsions described herein can be stable or unstable. The emulsions can be relatively stable and have minimal coalescence. Coalescence can occur when small droplets combine to form progressively larger ones. In some cases, less than 0.00001%, 0.00005%, 0.00010%, 0.00050%, 0.001%, 0.005%, 0.01%, 0.05%, 0.1%, 0.5%, 1%, 2%, 2.5%, 3%, 3.5%, 4%, 4.5%, 5%, 6%, 7%, 8%, 9%, or 10% of droplets generated from a droplet generator coalesce with other droplets. The emulsions can also have limited flocculation, a process by which the dispersed phase comes out of suspension in flakes.

Splitting a sample into small reaction volumes as described herein can enable the use of reduced amounts of reagents, thereby lowering the material cost of the analysis. Reducing sample complexity by partitioning also improves the dynamic range of detection because higher-abundance molecules are separated from low-abundance molecules in different compartments, thereby allowing lower-abundance molecules greater proportional access to reaction reagents, which in turn enhances the detection of lower-abundance molecules.

Droplets can be generated having an average diameter of about, less than, or more than, or at least 0.001, 0.01, 0.05, 0.1, 1, 5, 10, 20, 30, 40, 50, 60, 70, 80, 100, 120, 130, 140, 150, 160, 180, 200, 300, 400, or 500 microns. Droplets can have an average diameter of about 0.001 to about 500, about 0.01 to about 500, about 0.1 to about 500, about 0.1 to about 100, about 0.01 to about 100, or about 1 to about 100 microns. Microfluidic methods of producing emulsion droplets using microchannel cross-flow focusing or physical agitation are known to produce either monodisperse or polydisperse emulsions. The droplets can be monodisperse droplets. The droplets can be generated such that the size of the droplets does not vary by more than plus or minus 5% of the average size of the droplets. In some cases, the droplets are generated such that the size of the droplets does not vary by more than plus or minus 2% of the average size of the droplets. A droplet generator can generate a population of droplets from a single sample, wherein none of the droplets vary in size by more than plus or minus about, more than, less than, or at least 0.1%, 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% of the average size of the total population of droplets.

Higher mechanical stability can be useful for microfluidic manipulations and higher-shear fluidic processing (e.g., in microfluidic capillaries or through 90 degree turns, such as valves, in fluidic path). Pre- and post-thermally treated droplets or capsules can be mechanically stable to standard pipet manipulations and centrifugation.

A droplet can be formed by flowing an oil phase through an aqueous sample. The aqueous phase can comprise a buffered solution and reagents for performing a PCR reaction, including nucleotides, primers, probe(s) for fluorescent detection, template nucleic acids, DNA polymerase enzyme, and optionally, reverse transcriptase enzyme.

The aqueous phase can comprise a buffered solution and reagents for performing a PCR reaction without solid-state beads, such as magnetic-beads. The buffered solution can comprise about, more than, or less than, or at least 1, 5, 10, 15, 20, 25, 30, 50, 100, or 200 mM Tris. In some cases, the buffer concentration is about 1 to about 200, about 5 to about 200, about 10 to about 200, about 20 to about 200, about 10 to about 50, about 10 to about 30, about 15 to about 25, or about 25 to about 200 mM. In some cases, the concentration of potassium chloride can be about, more than, or less than, or at least 10, 20, 30, 40, 50, 60, 80, 100, 200 mM. The buffered solution can comprise about 15 mM Tris and 50 mM KCl. The nucleotides can comprise deoxyribonucleotide triphosphate molecules, including dATP, dCTP, dGTP, dTTP, in concentrations of about, more than, or less than, or at least 50, 100, 200, 300, 400, 500, 600, or 700 µM each. The concentration of each dNTP in a reaction can be about 50 to about 700, about 100 to about 300, or about 100 to about 400 µM. In some cases dUTP is added within the aqueous phase to a concentration of about, more than, or less than, or at least 50, 100, 200, 300, 400, 500, 600, or 700, 800, 900, or 1000 µM. The concentration of dUTP in a reaction can be about 50 to about 700, about 100 to about 300, or about 100 to about 400 µM. In some cases, magnesium chloride or magnesium acetate (MgCl₂) is added to the aqueous phase at a concentration of about, more than, less than, or at least 1.0, 2.0, 3.0, 4.0, or 5.0 mM. In some cases, the concentration of magnesium salt is about 1 to about 10, about 1 to about 5, or about 2 to about 5 mM. The concentration of MgCl₂ can be about 3.2mM. In some cases, magnesium acetate is used.

A non-specific blocking agent such as BSA or gelatin from bovine skin can be used, wherein the gelatin or BSA is present in a concentration range of about 0.1 to about 0.9% w/v. Other possible blocking agents can include betalactoglobulin, casein, dry milk, or other common blocking agents. In some cases, preferred concentrations of BSA and gelatin are about 0.1% w/v.

Primers for amplification within the aqueous phase can have a concentration of about, more than, less than, or at least 0.05, 0.1, 0.2, 0.3, 0.4, 0.5, 0.6, 0.7, 0.8, 0.9, 1.0, 1.2, 1.5, 1.7, or 2.0 µM. Primer concentration within the aqueous phase can be about 0.05 to about 2, about 0.1 to about 1.0, about 0.2 to about 1.0, about 0.3 to about 1.0, about 0.4 to about 1.0, or about 0.5 to about 1.0 µM. The concentration of primers can be about 0.5 µM. The aqueous phase can comprise one or more probes for fluorescent detection, at a concentration of about, more than, or less than, or at least 0.05, 0.1, 0.2, 0.3, 0.4, 0.5, 0.6, 0.7, 0.8, 0.9, 1.0, 1.2, 1.4, 1.6, 1.8, or 2.0 µM. The concentration of a fluorescent probe can be about 0.005 to about 2, about 0.5 to about 2, or about 1 to about 2 µM. The aqueous phase can comprise one or more probes for fluorescent detection, at a concentration of about 0.05 to about 2.0, about 0.1 to about 2.0, about 0.25 to about 2.0, about 0.5 to about 2.0, about 0.05 to about 1, about 0.1 to about 1, or about 0.1 to about 0.5 µM. The concentration of probes for fluorescent detection can be about 0.25 µM. Amenable ranges for target nucleic acid amounts in PCR are between about 1 pg and about 500 ng.

In some cases, the aqueous phase can also comprise additives including, but not limited to, non-specific background/blocking nucleic acids (e.g., salmon sperm DNA), biopreservatives (e.g. sodium azide), PCR enhancers (e.g. Betaine, Trehalose, etc.), and inhibitors (e.g. RNAse inhibitors).

In some cases, a non-ionic Ethylene Oxide/Propylene Oxide block copolymer is added to the aqueous phase in a concentration of about, more than, less than, or at least 0.1%, 0.2%, 0.3%, 0.4%, 0.5%, 0.6%, 0.7%, 0.8%, 0.9%, or 1.0%. Common biosurfactants include non-ionic surfactants such as Pluronic F-68, Tetronics, Zonyl FSN. Pluronic F-68 can be present at a concentration of about 0.5% w/v.

In some cases magnesium sulfate can be substituted for magnesium chloride, at similar concentrations. A wide range of common, commercial PCR buffers from varied vendors can be substituted for the buffered solution.

The oil phase can comprise a fluorinated base oil which can be additionally stabilized by combination with a fluorinated surfactant such as a perfluorinated polyether. In some cases, the base oil can be one or more of HFE 7500, FC-40, FC-43, FC-70, or other common fluorinated oil. In some cases, the anionic surfactant is Ammonium Krytox (Krytox-AM), the ammonium salt of Krytox FSH, or morpholino derivative of Krytox-FSH. Krytox-AS can be present at a concentration of about, more than, or less than, or at least 0.1%, 0.2%, 0.3%, 0.4%, 0.5%, 0.6%, 0.7%, 0.8%, 0.9%, 1.0%, 2.0%, 3.0%, or 4.0% w/w. In some cases, the concentration of Krytox-AS is about 1.8%. In other cases, the concentration of Krytox-AS is about 1.62%. Morpholino derivative of Krytox-FSH can be present at a concentration of about 0.1%, 0.2%, 0.3%, 0.4%, 0.5%, 0.6%, 0.7%, 0.8%, 0.9%, 1.0%, 2.0%, 3.0%, or 4.0% w/w. The concentration of morpholino derivative of Krytox-FSH can be about 1.8%. The concentration of morpholino derivative of Krytox-FSH can be about 1.62%.

The oil phase can further comprise an additive for tuning the oil properties, such as vapor pressure or viscosity or surface tension. Nonlimiting examples include perfluoro-octanol and 1H,1H,2H,2H-Perfluorodecanol. 1H,1H,2H,2H-Perfluorodecanol can be added to a concentration of about, more than, less than, or at least 0.05%, 0.06%, 0.07%, 0.08%, 0.09%, 1.00%, 1.25%, 1.50%, 1.75%, 2.00%, 2.25%, 2.50%, 2.75%, or 3.00% w/w. 1H,1H,2H,2H-Perfluorodecanol can be added to a concentration of about 0.18% w/w.

The emulsion can formulated to produce highly monodisperse droplets having a liquid-like interfacial film that can be converted by heating into microcapsules having a solid-like interfacial film; such microcapsules can behave as bioreactors able to retain their contents through a reaction process such as PCR amplification. The conversion to microcapsule form can occur upon heating. For example, such conversion can occur at a temperature of greater than 50, 60, 70, 80, 90, or 95 degrees Celsius. In some cases this heating occurs using a thermocycler. During the heating process, a fluid or mineral oil overlay can be used to prevent evaporation. Excess continuous phase oil may or may not be removed prior to heating. The biocompatible capsules can be resistant to coalescence and/or flocculation across a wide range of thermal and mechanical processing.

Following conversion, the capsules can be stored at about, more than, or less than, or at least 3, 4, 5, 6, 7, 8, 9, 10, 15, 20, 25, 30, 35, or 40 degrees Celsius. These capsules can be useful in biomedical applications, such as stable, digitized encapsulation of macromolecules, particularly aqueous biological fluids containing a mix of nucleic acids or protein, or both together; drug and vaccine delivery; biomolecular libraries; clinical imaging applications, and others.

The microcapsules can contain one or more polynucleotides and may resist coalescence, particularly at high temperatures. Accordingly, PCR amplification reactions can occur at a very high density (e.g., number of reactions per unit volume). In some cases, greater than 100,000, 500,000, 1,000,000, 1,500,000, 2,000,000, 2,500,000, 5,000,000, or 10,000,000 separate reactions can occur per ml. In some cases, the reactions occur in a single well, e.g., a well of a microtiter plate, without inter-mixing between reaction volumes. The microcapsules can also contain other components necessary to enable a PCR reaction to occur, e.g., primers, probes, dNTPs, DNA or RNA polymerases, etc. These capsules exhibit resistance to coalescence and flocculation across a wide range of thermal and mechanical processing.

In some cases, the droplet is generated using commercially available droplet generator, such as Bio-Rad QX100™ Droplet Generator. RT and the droplet PCR can be carried out using commercially available reagents and systems, and the droplet can be analyzed using a commercially available droplet reader, such as Bio-Rad QX100™ Droplet Reader.

### Digital Analysis

The methods provided herein are suitable for use with a digital analysis technique. The digital analysis can be digital polymerase chain reaction (digital PCR, DigitalPCR, dPCR, or dePCR). The dPCR can be droplet dPCR (ddPCR).

In general, dPCR can involve spatially isolating (or partitioning) individual polynucleotides from a sample and carrying out a polymerase chain reaction on each partition. The partition can be, e.g., a well (e.g., wells of a microwell plate), capillary, dispersed phase of an emulsion, a chamber (e.g, a chamber in an array of miniaturized chambers), a droplet, or a nucleic acid binding surface. The sample can be distributed so that each partition has 0 or 1 polynucleotides. After PCR amplification, the number of partitions with or without a PCR product can be enumerated. The total number of partitions can be about, more than, less than, or at least, 500, 1000, 2000, 3000, 4000, 5000, 6000, 7000, 8000, 9000, 10,000, 11,000, 12,000, 13,000, 14,000, 15,000, 16,000, 17,000, 18,000, 19,000, 20,000, 30,000, 40,000, 50,000, 60,000, 70,000, 80,000, 90,000, 100,000, 150,000, 200,000, 500,000, 750,000, 1,000,000, 2,500,000, 5,000,000, 7,500,000, 10,000,000, 25,000,000, 50,000,000, 75,000,000, or 100,000,000. In some cases, the total number of partitions is about 1000 to about 10,000, about 10,000 to about 100,000, about 100,000 to about 1,000,000, about 1,000,000 to about 10,000,000, or about 10,000,000 to about 100,000,000. Positive and negative droplets can be counted, and software can calculate a concentration of target RNA as copies per microliter.

Less than 0.00001, 0.00005, 0.00010, 0.00050, 0.001, 0.005, 0.01, 0.05, 0.1, 0.5, 1, 2, 2.5, 3, 3.5, 4, 4.5, 5, 6, 7, 8, 9, or 10 copies of target polynucleotide can detected. In some cases, less than 1, 2, 3, 4, 5, 6, 7, 8, 9, 10, 11, 12, 13, 14, 15, 20, 25, 30, 35, 40, 45, 50, 55, 60, 65, 70, 75, 80, 85, 90, 95, 100, 150, 200, 250, 300, 350, 400, 450, or 500 copies of a target polynucleotide are detected. In some cases, the droplets described herein are generated at a rate of greater than 1, 2, 3, 4, 5, 10, 50, 100, 200, 300, 400, 500, 600, 700, 800, 900, 1000, 2500, 5000, 10,000, 25,000, 50,000, 75,000, 100,000, 250,000, 500,000, or 1,000,000 droplets/second. In some cases, the droplets described herein are generated at a rate of about 1 to about 10, about 10 to about 100, about 100 to about 1000, about 1000 to about 10,000, about 10,000 to about 100,000, or about 100,000 to about 1,000,000 droplets/second.

An integrated, rapid, flow-through thermal cycler device can be used in the methods described herein. *See*, *e.g*., International Application No. PCT/US2009/005317, filed 9-23-2009. In such an integrated device, a capillary is wound around a cylinder that maintains 2, 3, or 4 temperature zones. As droplets flow through the capillary, they are subjected to different temperature zones to achieve thermal cycling. The small volume of each droplet results in an extremely fast temperature transition as the droplet enters each temperature zone.

A digital PCR device (e.g., droplet digital PCR device) for use with the methods, compositions, and kits described herein can detect multiple signals (see e.g. US Provisional Patent Application No. 61/454,373, filed March 18, 2011, herein incorporated by reference in its entirety).

### Droplet RT-PCR

In another aspect, the methods and compositions provided herein allow for performance of one step RT-PCR in a single droplet.

A one-pot RT-PCR provided herein can enable the performance of RT-PCR in droplets, which further enables the performance of digital RT-PCR in droplets.

A common method to measure RNA in a sample is to use real-time RT-PCR. In real-time PCR, the threshold cycle (the number of cycles of amplification required before fluorescence signals first become clearly visible) is linearly proportional to the logarithm of the number of template molecules initially present in the sample. However, when the sample possesses only a few target molecules, that relationship is subject to statistical uncertainties during the early stages of PCR. A sample containing only eight template molecules may behave like a sample possessing twenty-five molecules or like a sample containing only two molecules. Sometimes researchers can overcome this statistical limitation by analyzing the sample many times and then calculating the mean threshold cycle as a measure of target-copy number. This defect is overcome by digital RT-PCR. In digital RT-PCR, a sample containing RNA from a single cell can be divided into a large number of partitions, so that each partition is likely to receive only a single template molecule. Fluorescent probes (such as molecular beacons or TaqMan probes) can be used to light up the wells that contain amplified template molecules. The number of illuminated wells can provide a direct measure of the number of target molecules in the sample.

In some cases, a primer/probe is used; in some cases, the primer/probe is a Scorpion™ probe. A Scorpion™ probe can provide a FRET-based stem-loop detection mechanism similar to Molecular Beacon, except that the probe also has a segment attached that serves as an amplification primer (see e.g., Whitcombe et al. Nat Biotechnol. 1999, August 17(8): 804-7; U.S. Pat. No. 6,326,145). A Scorpion™ probe can maintain a stem-loop configuration in the unhybridized state with the fluorophore thereby quenched. A Scorpion™ probe can have a longer multi-component structure, e.g., a 5' fluorophore, then a target-specific stem-loop section, then a quencher (e.g., Black Hole Quencher™), then a blocker (e.g., hexethelene glycol (HEG)), and finally a 3' primer sequence. The blocker can prevent reverse extension of the product onto the probe. After primer extension occurs, the Scorpion™ probe can be attached to the terminal end of the amplicon. When denaturation occurs again, followed by annealing, the loop segment of the probe can preferentially bind to its long complementary segment on the attached template, thereby opening the stem-loop structure and releasing fluorescence. Alternatively, the stem-loop structure can be cut into two units with one unit having four components, e.g., a 5' fluorophore, a target specific segment, a blocker and a primer, and the other unit having the quencher and a probe segment.

In some cases, a primer/probe is a Sunrise™ probe. A Sunrise™ probe can comprise a primer attached to a hairpin probe that is extended during amplification. This arrangement can separate the internal quencher label from the 5' terminal fluorophore (Nazarenko et al., Nucl. Acids Res. 1997, 25: 2516-2521).

Originally, digital PCR was performed in 96- or 384-well format and therefore had a dynamic range of 0-100 target molecules. The dynamic range of these assays has been improved by the introduction of assay platforms possessing thousands of individual wells. In one format, there are 3,000 wells etched into the surface of a glass slide, each with a volume of a few nanoliters. A sample can be distributed among the wells, and real-time PCR can be performed. In a related format, 1,200 chambers are created in a microfluidic device by the intersection of rows and columns of channels and valves.

In a format termed BEAMing (Beads, Emulsions, Amplification, and Magnetics), the sample is distributed into hundreds of thousands of microdroplets in a thermostable water-oil emulsion. Each microdroplet can also contain a paramagnetic bead that binds to the amplified DNA and becomes labeled by fluorescent reporters associated with the amplified DNA. At the end of the amplification, the beads are separated from the emulsion and counted by a fluorescence-activated cell sorter. In another format, the sample is diluted in a gel, and PCR is performed within the gel. Since the gel limits the diffusion of the amplicons during PCR, molecular colonies form at the positions of the original target molecules, and the number colonies indicates the number of targets that were in the original sample.

Described herein are methods and compositions for carrying out digital RT-PCR in droplets without beads or gel, and the digital droplet PCR can be carried out through a one-pot reaction mix.

In some cases, the method for reverse transcribing and amplifying an RNA in a single droplet comprises: (a) contacting an RNA with a reaction mixture comprising a first enzyme comprising reverse transcriptase activity, under a condition suitable for reverse transcription reaction, thereby reverse transcribing the RNA into a DNA; and (b) amplifying the DNA in the reaction mixture with a second enzyme having DNA-dependent DNA polymerase activity, wherein the reaction mixture is contained in a droplet. In some cases, there is no need to manipulate the droplets between the RT reaction and PCR reaction, such as adding or removing one or more components to the droplets. In some cases, there is also no need to use beads in the droplets, or form different droplets, or to perform the RT reaction and PCR reaction separately. In some cases, droplets do not contact a bead or gel. In some cases, a droplet does contain a bead or gel.

In some cases, a reaction mixture comprises manganese and magnesium. In some cases, the magnesium is inaccessible to the first enzyme during at least part of the duration of the reverse transcription reaction. In some cases, the magnesium is accessible to the second enzyme during the amplification reaction.

In some cases, droplets have reaction volumes that range from about 1 pL to about 100 nL, or about 1 pL to about 10 nl, or about 10 pL to about 1 nL. In some cases, the reaction mixture is contained in a plurality of droplets.

In some cases, droplet merging can be used to merge a second droplet containing magnesium with a first droplet containing a reverse transcription reaction mix after a reverse transcription reaction is completed in the first droplet. Reverse transcription can be performed in the first droplet to generate cDNA, and after the reverse transcription reaction, the droplet containing the reverse transcription mix and generated cDNA can be merged with the second droplet containing magnesium, and optionally a second enzyme (e.g., DNA dependent DNA polymerase), for performing PCR. In some cases, a droplet containing a reverse transcription reaction mix (e.g., reverse transcriptase, manganese) is merged with a droplet containing magnesium prior to a reverse transcription reaction.

### II. Kits

Described here are kits for reverse transcribing and amplifying an RNA. A kit can comprise a first solution comprising: (i) a buffer; (ii) a monovalent cation; (iii) a magnesium salt; (iv) dATP, dCTP, dGTP, and dTTP, or analogs thereof; (v) a first enzyme comprising reverse transcriptase activity; and (vi) a second enzyme comprising DNA-dependent DNA polymerase activity, wherein the reaction mixture does not contain manganese.

In some cases, the buffer comprises Tris or Tricine. The pH of the buffer can be from about 7 to about 8.8, or about 8.3. The pH of a reaction mix can from about 5 to about 10, from about 6 to about 9, from about 7 to about 8, or from about 7 to about 10.

In some cases, the first solution comprises a magnesium salt selected from the group consisting of MgCl₂, MgSO₄, and Mg(OAc)₂. The concentration of magnesium can be from about 0.5 mM to about 2 mM, or about 1 mM.

In some cases the first solution comprises a dNTP analogue (e.g., 7-deaza-dGTP).

In some cases the second enzyme comprises a hot-start polymerase.

In some cases the monovalent cation is K⁺ or Na⁺.

In some cases the kit further comprises a second solution comprising a manganese salt selected from the group consisting of MnCl₂, MnSO₄, and Mn(OAc)₂.

In another aspect, provided herein is a method for preparing a reaction mixture, comprising: (a) providing a first solution comprising: (i) a buffer, e.g., Tris-Cl, e.g., at pH 8.3; (ii) a monovalent cation (e.g., K⁺ or Na⁺); (iii) a magnesium salt, e.g., MgCl₂, MgSO₄, or Mg(OAc)₂.; (iv) dATP, dCTP, dGTP, and dTTP, and/or analogs thereof (e.g. 7-deaza-dGTP), and optionally dUTP; (v) a first enzyme comprising reverse transcriptase activity, e.g., a thermostable polymerase known in the art or provided herein; (vi) a second enzyme comprising DNA-dependent DNA polymerase activity, e.g., a hot-start polymerase; and (vii) one or more pairs of primers for amplifying a target sequence of an RNA, wherein the first solution does not contain manganese; and (b) mixing the first solution with a second solution comprising manganese, e.g., MnCl₂, MnSO₄,or Mn(OAc)₂.

In a further aspect, provided herein is a method for reverse transcribing and amplifying an RNA, the method comprising: (a) contacting an RNA comprising a target sequence with a reaction mixture prepared according to a method provided herein; and (b) amplifying the DNA in the same reaction mixture with a second enzyme. In general, the magnesium is inaccessible to the first enzyme during at least part of the duration of the reverse transcription reaction, and the magnesium is accessible to the second enzyme during the amplification reaction.

### III. Applications

The RT-PCR provided herein can be used in any applications known in the art that requires the measurement of RNA concentration or detection of the presence of an RNA in a sample, such as measuring the expression level of mRNA in a sample, which is useful in diagnosis and drug development, or determining the presence of a RNA of an RNA virus, which is useful in infectious diseases diagnosis and treatment.

### RNA Viruses

In some cases, the methods described herein are used to amplify RNA from one or more RNA viruses. The RNA virus can be a bunyavirus, flavivirus, monoegavirales, nidovirales, orthomyxoviridae, picronavirales, picornavirus, potyviridae, rotavirus, togvirus, or tymovirales.

A bunyavirus can be an Akabane virus, Andes virus, Bwamba Fever virus, California encephalitis virus, Crimean-Congo hemorrhagic fever virus, Ganjam virus, hantavirus, Heartland virus, Impatiens necrotic spot virus, Kupe virus, La Crosse encephalitis virus, Nairovirus, Orthobunyavirus, Phlebovirus, Playa de Oro virus, Puumala virus, Qalyub virus, Rift Valley fever virus, Schmallenberg virus, Seoul virus, Sever fever with thromobytopenia syndrome virus, Tete virus, Toscana virus, Tospovirus, or Sin Nombre virus.

A flavivirus can be alkhurma virus, bovine virus, classical swine fever virus, deer tick virus, dengue virus, gadgets gully virus, GB virus C, hepacivirus, Hepatitis C virus, Japanese encephalitis virus, Kadam virus, Kunjin virus, Kyasanur forest disease virus, Langat virus, Murray Valley encephalitis virus, Omsk hemorrhagic fever virus, pestivirus, powassan virus, Saint Louis encephalitis virus, Tick-borne encephalitis virus, Usutu virus, West Nile virus, Yellow fever virus, or Zika virus.

A Mononegavirales can be an Australian bat lyssavirus, Avian paramyxovirus, Avian pneumovirus, Avulavirus, Barley yellow striate mosaic virus, Bas-Congo virus, Blue Eye Disease virus, Borna disease virus, Bornavirus, Broccoli necrotic yellows virus, Bundibugyo ebolavirus, Bundibugyo virus, Canine distemper virus, Chandipura virus, Duvenhage virus, Ebola virus, Filoviridae, Henipavirus, Human parainfluenza viruses, Human respiratory syncytial virus, Infectious hematopoietic necrosis virus, Lagos bat virus, Lettuce necrotic yellows virus, Lloviu cuevavirus, Lloviu virus, Lyssavirus, Maize mosaic virus, Marburg virus, Measles virus, Menangle virus, Metapneumovirus, Mokola virus, Morbillivirus, Mumps virus, Newcastle disease virus, Novirhabdovirus, Ovine rinderpest virus, Paramyxovirus, Phocine distemper virus, Potato yellow dwarf virus, Rabies virus, Ravn virus, Reston virus, Rhabdoviridae, Rinderpest virus, Rubulavirus, Sendai virus, Shimoni bat virus, Sigma viruses, Snakehead rhabdovirus, Sowthistle yellow vein virus, Strawberry crinkle virus, Sudan ebolavirus, Sudan virus, Taï Forest ebolavirus, Taï Forest virus, Tioman virus, Vesicular stomatitis virus or a Viral hemorrhagic septicemia virus.

A Nidovirales can be an Arterivirus, Avian infectious bronchitis virus, Canine coronavirus, Coronaviridae, Coronavirus, Feline infectious peritonitis virus, HCoV-EMC, Human Coronavirus NL63, Mouse hepatitis virus, Okavirus, Porcine reproductive and respiratory syndrome virus, Roniviridae, SARS coronavirus, Turkey coronavirus or a Yellowhead disease.

An Orthomyxoviridae can be a Dhori virus, Infectious salmon anemia virus, Influenza A virus, Influenzavirus B, Influenzavirus C or a Thogotovirus.

A Picornavirales can be a Cheravirus, Cherry leaf roll virus, Cherry rasp leaf virus, Cowpea mosaic virus, Nepovirus, Sadwavirus, Satsuma dwarf virus, Secoviridae, Strawberry latent ringspot virus or a Strawberry mottle virus.

A Picornavirus can be an Aphthovirus, Bovine enterovirus, Cardiovirus, Coxsackie A virus, Coxsackie B virus, Coxsackie B4 virus, Coxsackievirus, Cricket paralysis virus, Echovirus, Encephalomyocarditis virus, Enterovirus, Erbovirus, Enterovirus 71, Foot-and-mouth disease virus, Hand, foot and mouth disease virus, Hepatitis A virus, Hepatovirus, Kakugo virus, Kobuvirus, Ljungan virus, Mengovirus, Parechovirus, Poliovirus, Rhinovirus, Saffold virus, Seneca Valley virus-001, SMEDI virus, Swine vesicular disease virus, Teschovirus, Theilovirus or a Turkey hepatitis virus.

A Potyviridae can be an Abaca bract mosaic virus, Agropyron mosaic virus, Apium virus Y, Asparagus virus 1, Banana bract mosaic virus, Bean yellow mosaic virus, Bidens mottle virus, Bramble yellow mosaic virus, Carrot thin leaf virus, Cassava brown streak virus , Celery mosaic virus, Chickpea bushy dwarf virus, Chickpea distortion mosaic virus, Chickpea filiform virus, Chilli veinal mottle virus, Clover yellow vein virus, Commelina mosaic virus, Groundnut eyespot virus, Guinea grass mosaic virus, Ipomovirus, Johnsongrass mosaic virus, Kalanchoë mosaic virus, Lettuce mosaic virus, Maize dwarf mosaic virus, Malva vein clearing virus, Oat mosaic virus, Oat necrotic mottle virus, Papaya ringspot virus, Passionfruit woodiness virus, Pea seed-borne mosaic virus, Peanut green mosaic virus, Peanut mottle virus, Pepper mottle virus, Plum pox virus, Potato virus A, Potato virus V, Potato virus Y, Potyvirus, Primula mosaic virus, Primula mottle virus, Sorghum mosaic virus, Soybean mosaic virus, Sugarcane mosaic virus, Sweet potato feathery mottle virus, Sweet potato latent virus, Sweet potato mild mottle virus, Sweet potato yellow dwarf virus, Tobacco etch virus, Tobacco vein mottling virus, Tradescantia mosaic virus, Turnip mosaic virus, Wheat spindle streak mosaic virus, Wheat streak mosaic virus, Wheat yellow mosaic virus, Wild potato mosaic virus or a Zucchini yellow mosaic virus.

A Rotavirus can be NSP1 (rotavirus), NSP2 (rotavirus), NSP3 (rotavirus), NSP4 (rotavirus), NSP5 (rotavirus), NSP6 (rotavirus) or a Rotavirus enteritis.

A Togavirus can be an Alphavirus, Babanki virus, Chikungunya, Eastern equine encephalitis virus, Everglades virus, Highlands J virus, O'nyong'nyong virus, Ross River virus, Rubella virus, Sagiyama virus, Semliki Forest virus, Sindbis virus, Una virus or a Venezuelan equine encephalitis virus.

A Tymovirales can be an Alphaflexiviridae, Betaflexiviridae, Carlavirus, Flexiviridae, Gammaflexiviridae, Potato virus X, Potexvirus, Trichovirus or a Tymoviridae.

Methods of studying RNA viruses are described, e.g., in U.S. Patent Application Publication Nos. 20120270321 and 20120231539, and U.S. Patent Nos. 6723559, 8283310, and 8283329.

### Pathogens

The present methods, compositions, or kits can be used to detect or monitor pathogenic organisms (*e.g.*, *Staphylococcus aureus*, *Methicillin resistant Staphylococcus aureus* (MRSA), *Methicillin-Sensitive Staphylococcus Aureus* (MSSA), *Mycobacterium tuberculosis* (MTB), multi-drug resistant strains of Mycobacterium tuberculosis). In some cases, the methods, compositions, or kits can be used to detect *Escherichia coli*, *Salmonella*, *Shigella*, *Klebsiella*, *Pseudomonas*, *Pseudomonas aeruginosa*, *Listeria monocytogenes*, *Mycobacterium aviumintracellulare*, *Yersinia*, *Francisella*, *Pasteurella*, *Brucella*, *Clostridia*, *Bordetella pertussis*, *Bacteroides*, *Staphylococcus aureus, Streptococcus pneumonia*, *B-Hemolytic strep*., *Corynebacteria*, *Legionella*, *Mycoplasma*, *Ureaplasma*, *Chlamydia*, *Neisseria gonorrhea*, *Neisseria meningitides*, *Hemophilus influenza*, *Enterococcus faecalis*, *Proteus vulgaris*, *Proteus mirabilis*, *Helicobacter pylori*, *Treponema palladium*, *Borrelia burgdorferi*, *Borrelia recurrentis*, *Rickettsial pathogens*, *Nocardia*, and *Acitnomycetes.* Fungal infectious agents which can be detected by the present methods, compositions, or kits include *Cryptococcus neoformans*, *Blastomyces dermatitidis*, *Histoplasma capsulatum*, *Coccidioides immitis*, *Paracoccidioides brasiliensis*, *Candida albicans*, *Aspergillus fumigautus*, *Phycomycetes* (*Rhizopus*), *Sporothrix schenckii*, *Chromomycosis*, and *Maduromycosis*. Viral infections, or free virus, which can be detected and/or monitored by the present methods and compositions include human immunodeficiency virus (HIV), HIV-1, HIV-2, human T-cell lymphocytotrophic virus, hepatitis viruses (e.g., Hepatitis B Virus and Hepatitis C Virus), Epstein-Barr Virus, cytomegalovirus, human papillomaviruses, orthomyxo viruses, paramyxo viruses, adenoviruses, corona viruses, rhabdo viruses, polio viruses, toga viruses, bunya viruses, arena viruses, rubella viruses, and reo viruses. Parasitic agents which can be detected and/or monitored by the present methods, compositions, and kits include malarial parasites, *Plasmodium falciparum*, *Plasmodium malaria*, *Plasmodium vivax*, *Plasmodium ovale*, *P. knowlesi*, *Onchoverva volvulus*, *Leishmania*, *Trypanosoma spp*., *Schistosoma spp*., *Entamoeba histolytica*, *Cryptosporidum*, *Giardia spp*., *Trichimonas spp*., *Balatidium coli*, *Wuchereria bancrofti*, *Toxoplasma spp*., *Enterobius vermicularis*, *Ascaris lumbricoides*, *Trichuris trichiura*, *Dracunculus medinesis*, *trematodes*, *Diphyllobothrium latum*, *Taenia spp*., *Pneumocystis carinii*, and *Necator americanis.*

The present methods and compositions are also useful for detection of drug resistance. For example, vancomycin-resistant *Enterococcus faecium*, *methicillin-resistant Staphylococcus aureus*, penicillin-resistant *Streptococcus pneumoniae*, multi-drug resistant *Mycobacterium tuberculosis*, and AZT-resistant human immunodeficiency virus can all be identified with the present methods, compositions, or kits.

Means of detecting pathogens are described, e.g., in EP Patent Application No. EP2152871 and U.S. Patent Application Publication Nos. 20120231445, 20120308999, 20120283135, and US20120201851.

### Bioterrorism/forensics/epidemiology and other uses

The methods, compositions, and kits provided herein can be used to detect or monitor cells and viruses potentially associated with a bioterrorist or biowarfare attack, including, but not limited to: Botulinum neurotoxin producing species of *Clostridium*, *Cercopithecine herpesvirus* 1 (Herpes B virus), *Clostridium perfringens*, *Coccidioides posadasii*/*Coccidioides immitis*, *Coxiella burnetii*, Crimean-Congo haemorrhagic fever virus, Eastern Equine Encephalitis virus, Ebola virus, Francisella tularensis, Lassa fever virus, Marburg virus, Monkeypox virus, reconstructed replication competent forms of the 1918 flu pandemic containing any portion of the coding regions of all eight gene segments (reconstructed 1918 Influenza virus), influenza A H1N1, influenza A H5N1, influenza A H3N2, *Rickettsia prowazekii*, *Rickettsia rickettsii*, South American Haemorrhagic Fever viruses, Guanarito virus, Junin virus, Machupo virus, Sabia virus, Tick-borne encephalitis complex (flavi) viruses, Central European Tick-borne encephalitis Far Eastern Tick-borne encephalitis, Kyasanur Forest disease, Omsk Hemorrhagic Fever Russian Spring and Summer encephalitis, Variola major virus (Smallpox virus), Variola minor virus (Alastrim), Yersinia pestis, and/or anthrax (*Bacillus anthracis*).

In some cases, the methods, compositions, and kits described herein can be used for forensics or epidemiology. The methods, compositions, and kits described herein can be used to monitor an epidemic or a pandemic, e.g., an influenza pandemic. In some cases, the methods, compositions, and kits described herein can be used in archaeology.

### Conditions

The methods, compositions, and kits described herein can be used to detect, diagnose, prognose, and/or monitor one or more cancers or conditions. The condition or cancer can include, for example, acute myeloid leukemia; bladder cancer, including upper tract tumors and urothelial carcinoma of the prostate; bone cancer, including chondrosarcoma, Ewing's sarcoma, and osteosarcoma; breast cancer, including noninvasive, invasive, phyllodes tumor, Paget's disease, and breast cancer during pregnancy; central nervous system cancers, adult low-grade infiltrative supratentorial astrocytoma/oligodendroglioma, adult intracranial ependymoma, anaplastic astrocytoma/anaplastic oligodendroglioma/glioblastoma multiforme, limited (1-3) metastatic lesions, multiple (>3) metastatic lesions, carcinomatous lymphomatous meningitis, nonimmunosuppressed primary CNS lymphoma, and metastatic spine tumors; cervical cancer; chronic myelogenous leukemia (CML); colon cancer, rectal cancer, anal carcinoma; esophageal cancer; gastric (stomach) cancer; head and neck cancers, including ethmoid sinus tumors, maxillary sinus tumors, salivary gland tumors, cancer of the lip, cancer of the oral cavity, cancer of the oropharynx, cancer of the hypopharynx, occult primary, cancer of the glottic larynx, cancer of the supraglottic larynx, cancer of the nasopharynx, and advanced head and neck cancer; hepatobiliary cancers, including hepatocellular carcinoma, gallbladder cancer, intrahepatic cholangiocarcinoma, and extrahepatic cholangiocarcinoma; Hodgkin disease/lymphoma; kidney cancer; melanoma; multiple myeloma, systemic light chain amyloidosis, Waldenstrom's macroglobulinemia; myelodysplastic syndromes; neuroendocrine tumors, including multiple endocrine neoplasia, type 1, multiple endocrine neoplasia, type 2, carcinoid tumors, islet cell tumors, pheochromocytoma, poorly differentiated/small cell/atypical lung carcinoids; Non-Hodgkin's Lymphomas, including chronic lymphocytic leukemia/small lymphocytic lymphoma, follicular lymphoma, marginal zone lymphoma, mantle cell lymphoma, diffuse large B-Cell lymphoma, Burkitt's lymphoma, lymphoblastic lymphoma, AIDS-Related B-Cell lymphoma, peripheral T-Cell lymphoma, and mycosis fungoides/Sezary Syndrome; non-melanoma skin cancers, including basal and squamous cell skin cancers, dermatofibrosarcoma protuberans, Merkel cell carcinoma; non-small cell lung cancer (NSCLC), including thymic malignancies; occult primary; ovarian cancer, including epithelial ovarian cancer, borderline epithelial ovarian cancer (Low Malignant Potential), and less common ovarian histologies; pancreatic adenocarcinoma; prostate cancer; small cell lung cancer and lung neuroendocrine tumors; soft tissue sarcoma, including soft-tissue extremity, retroperitoneal, intra-abdominal sarcoma, and desmoid; testicular cancer; thymic malignancies, including thyroid carcinoma, nodule evaluation, papillary carcinoma, follicular carcinoma, Hiirthle cell neoplasm, medullary carcinoma, and anaplastic carcinoma; uterine neoplasms, including endometrial cancer and uterine sarcoma.

Means of studying cancer are described, e.g., in U.S. Patent No. 6869760 and U.S. Patent Application Publication No. 20070178461.

The methods, compositions, and kits described herein can be used to detect, diagnose, prognose, or monitor an autoimmune disease. The autoimmune disease can be, e.g., systemic lupus erythematosus, multiple sclerosis, rheumatoid arthritis, or Ankylosing Spondylitis. Methods of investigating autoimmune diseases are described, e.g., in U.S. Patent Nos. 5641864 and 6617171.

In some cases, the methods, compositions, and kits described herein can be used to detect, diagnose, prognose, or monitor a neurological or neurocognitive condition. The neurological or neurocognitive condition can be a neurological disorder listed on the National Institute of Neurological Disorders and Stroke webpage (www.ninds.nih.gov/disorders/disorder_index.htm). In some cases, the subject can have a sign or symptom. The neurological or neurocognitive condition, can be, e.g., abarognosis (e.g., loss of the ability to detect the weight of an object held in the hand or to discern the difference in weight between two objects), acid lipase disease, acid maltase deficiency, acquired epileptiform aphasia, absence of the septum pellucidum, acute disseminated encephalomyelitis, adie's pupil, Adie's syndrome, adrenoleukodystrophy, agenesis of the corpus callosum, agnosia, Aicardi syndrome, Aicardi-Goutieres syndrome disorder, AIDS - neurological complications, akathisia, alcohol related disorders, Alexander disease, Alien hand syndrome (anarchic hand), allochiria, Alpers' disease, altitude sickness, alternating hemiplegia, Alzheimer's disease, amyotrophic lateral sclerosis, anencephaly, aneurysm, Angelman syndrome, angiomatosis, anoxia, Antiphospholipid syndrome, aphasia, apraxia, arachnoid cysts, arachnoiditis, arnold-chiari malformation, Asperger syndrome, arteriovenous malformation, ataxia, ataxias and cerebellar or spinocerebellar degeneration, ataxia telangiectasia, atrial fibrillation, stroke, attention deficit hyperactivity disorder, auditory processing disorder, autism, autonomic dysfunction, back pain, Barth syndrome, Batten disease, becker's myotonia, Behcet's disease, bell's palsy, benign essential blepharospasm, benign focal amyotrophy, benign intracranial hypertension, Bernhardt-Roth syndrome, bilateral frontoparietal polymicrogyria, Binswanger's disease, blepharospasm, Bloch-Sulzberger syndrome, brachial plexus birth injuries, brachial plexus injury, Bradbury-Eggleston syndrome, brain or spinal tumor, brain abscess, brain aneurysm, brain damage, brain injury, brain tumor, Brown-Séquard syndrome, bulbospinal muscular atrophy, CADASIL (cerebral autosomal dominant arteriopathy subcortical infarcts and leukoencephalopathy), Canavan disease, Carpal tunnel syndrome, causalgia, cavernomas, cavernous angioma, cavernous malformation, Central cervical cord Syndrome, Central cord syndrome, Central pain syndrome, central pontine myelinolysis, centronuclear myopathy, cephalic disorder, ceramidase deficiency, cerebellar degeneration, cerebellar hypoplasia, cerebral aneurysm, cerebral arteriosclerosis, cerebral atrophy, cerebral beriberi, cerebral cavernous malformation, cerebral gigantism, cerebral hypoxia, cerebral palsy, cerebral vasculitis, Cerebro-Oculo-Facio-Skeletal syndrome (COFS), cervical spinal stenosis, Charcot-Marie-Tooth disease, chiari malformation, Cholesterol ester storage disease, chorea, choreoacanthocytosis, Chronic fatigue syndrome, chronic inflammatory demyelinating polyneuropathy (CIDP), chronic orthostatic intolerance, chronic pain, Cockayne syndrome type II, Coffin-Lowry syndrome, colpocephaly, coma, Complex regional pain syndrome, compression neuropathy, concussion, congenital facial diplegia, congenital myasthenia, congenital myopathy, congenital vascular cavernous malformations, corticobasal degeneration, cranial arteritis, craniosynostosis, cree encephalitis, Creutzfeldt-Jakob disease, cumulative trauma disorders, Cushing's syndrome, Cytomegalic inclusion body disease (CIBD), cytomegalovirus infection, Dancing eyes-dancing feet syndrome (opsoclonus myoclonus syndrome), Dandy-Walker syndrome (DWS), Dawson disease, decompression sickness, De morsier's syndrome, dejerine-klumpke palsy, Dejerine-Sottas disease, Delayed sleep phase syndrome, dementia, dementia - multi-infarct, dementia - semantic, dementia - subcortical, dementia with lewy bodies, dentate cerebellar ataxia, dentatorubral atrophy, depression, dermatomyositis, developmental dyspraxia, Devic's syndrome, diabetes, diabetic neuropathy, diffuse sclerosis, Dravet syndrome, dysautonomia, dyscalculia, dysgraphia, dyslexia, dysphagia, dyspraxia, dyssynergia cerebellaris myoclonica, dyssynergia cerebellaris progressiva, dystonia, dystonias, Early infantile epileptic, Empty sella syndrome, encephalitis, encephalitis lethargica, encephalocele, encephalopathy, encephalopathy (familial infantile), encephalotrigeminal angiomatosis, encopresis, epilepsy, epileptic hemiplegia, erb's palsy, erb-duchenne and dejerine-klumpke palsies, erythromelalgia, essential tremor, extrapontine myelinolysis, Fabry's disease, Fahr's syndrome, fainting, familial dysautonomia, familial hemangioma, familial idiopathic basal ganglia calcification, familial periodic paralyses, familial spastic paralysis, Farber's disease, febrile seizures, fibromuscular dysplasia, fibromyalgia, Fisher syndrome, floppy infant syndrome, foot drop, Foville's syndrome, friedreich's ataxia, frontotemporal dementia, Gaucher's disease, generalized gangliosidoses, Gerstmann's syndrome, Gerstmann-Straussler-Scheinker disease, giant axonal neuropathy, giant cell arteritis, Giant cell inclusion disease, globoid cell leukodystrophy, glossopharyngeal neuralgia, Glycogen storage disease, gray matter heterotopia, Guillain-Barré syndrome, Hallervorden-Spatz disease, head injury, headache, hemicrania continua, hemifacial spasm, hemiplegia alterans, hereditary neuropathies, hereditary spastic paraplegia, heredopathia atactica polyneuritiformis, herpes zoster, herpes zoster oticus, Hirayama syndrome, Holmes-Adie syndrome, holoprosencephaly, HTLV-1 associated myelopathy, HIV infection, Hughes syndrome, Huntington's disease, hydranencephaly, hydrocephalus, hydrocephalus - normal pressure, hydromyelia, hypercortisolism, hypersomnia, hypertension, hypertonia, hypotonia, hypoxia, immune-mediated encephalomyelitis, inclusion body myositis, incontinentia pigmenti, infantile hypotonia, infantile neuroaxonal dystrophy, Infantile phytanic acid storage disease, Infantile refsum disease, infantile spasms, inflammatory myopathy, inflammatory myopathies, iniencephaly, intestinal lipodystrophy, intracranial cyst, intracranial hypertension, Isaac's syndrome, Joubert syndrome, Karak syndrome, Kearns-Sayre syndrome, Kennedy disease, Kinsbourne syndrome, Kleine-Levin syndrome, Klippel feil syndrome, Klippel-Trenaunay syndrome (KTS), Klüver-Bucy syndrome, Korsakoffs amnesic syndrome, Krabbe disease, Kugelberg-Welander disease, kuru, Lafora disease, lambert-eaton myasthenic syndrome, Landau-Kleffner syndrome, lateral femoral cutaneous nerve entrapment, Lateral medullary (wallenberg) syndrome, learning disabilities, Leigh's disease, Lennox-Gastaut syndrome, Lesch-Nyhan syndrome, leukodystrophy, Levine-Critchley syndrome, lewy body dementia, Lipid storage diseases, lipoid proteinosis, lissencephaly, Locked-In syndrome, Lou Gehrig's, lumbar disc disease, lumbar spinal stenosis, lupus - neurological sequelae, lyme disease - neurological sequelae, Machado-Joseph disease (spinocerebellar ataxia type 3), macrencephaly, macropsia, megalencephaly, Melkersson-Rosenthal syndrome, Menieres disease, meningitis, meningitis and encephalitis, Menkes disease, meralgia paresthetica, metachromatic leukodystrophy, metabolic disorders, microcephaly, micropsia, migraine, Miller fisher syndrome, mini-stroke (transient ischemic attack), misophonia, mitochondrial myopathy, Mobius syndrome, Moebius syndrome, monomelic amyotrophy, mood disorder, Motor neurone disease, motor skills disorder, Moyamoya disease, mucolipidoses, mucopolysaccharidoses, multi-infarct dementia, multifocal motor neuropathy, multiple sclerosis, multiple system atrophy, multiple system atrophy with orthostatic hypotension, muscular dystrophy, myalgic encephalomyelitis, myasthenia - congenital, myasthenia gravis, myelinoclastic diffuse sclerosis, myoclonic encephalopathy of infants, myoclonus, myopathy, myopathy - congenital, myopathy - thyrotoxic, myotonia, myotonia congenita, myotubular myopathy, narcolepsy, neuroacanthocytosis, neurodegeneration with brain iron accumulation, neurofibromatosis, Neuroleptic malignant syndrome, neurological complications of AIDS, neurological complications of lyme disease, neurological consequences of cytomegalovirus infection, neurological manifestations of AIDS, neurological manifestations of pompe disease, neurological sequelae of lupus, neuromyelitis optica, neuromyotonia, neuronal ceroid lipofuscinosis, neuronal migration disorders, neuropathy - hereditary, neurosarcoidosis, neurosyphilis, neurotoxicity, neurotoxic insult, nevus cavernosus, Niemann-pick disease, Non 24-hour sleep-wake syndrome, nonverbal learning disorder, normal pressure hydrocephalus, O'Sullivan-McLeod syndrome, occipital neuralgia, occult spinal dysraphism sequence, Ohtahara syndrome, olivopontocerebellar atrophy, opsoclonus myoclonus, Opsoclonus myoclonus syndrome, optic neuritis, orthostatic hypotension, Overuse syndrome, chronic pain, palinopsia, panic disorder, pantothenate kinase-associated neurodegeneration, paramyotonia congenita, Paraneoplastic diseases, paresthesia, Parkinson's disease, paroxysmal attacks, paroxysmal choreoathetosis, paroxysmal hemicrania, Parry-Romberg syndrome, Pelizaeus-Merzbacher disease, Pena shokeir II syndrome, perineural cysts, periodic paralyses, peripheral neuropathy, periventricular leukomalacia, persistent vegetative state, pervasive developmental disorders, photic sneeze reflex, Phytanic acid storage disease, Pick's disease, pinched nerve, Piriformis syndrome, pituitary tumors, PMG, polio, polymicrogyria, polymyositis, Pompe disease, porencephaly, Post-polio syndrome, postherpetic neuralgia (PHN), postinfectious encephalomyelitis, postural hypotension, Postural orthostatic tachycardia syndrome, Postural tachycardia syndrome, Prader-Willi syndrome, primary dentatum atrophy, primary lateral sclerosis, primary progressive aphasia, Prion diseases, progressive hemifacial atrophy, progressive locomotor ataxia, progressive multifocal leukoencephalopathy, progressive sclerosing poliodystrophy, progressive supranuclear palsy, prosopagnosia, Pseudo-Torch syndrome, Pseudotoxoplasmosis syndrome, pseudotumor cerebri, Rabies, Ramsay hunt syndrome type I, Ramsay hunt syndrome type II, Ramsay hunt syndrome type III, Rasmussen's encephalitis, Reflex neurovascular dystrophy, Reflex sympathetic dystrophy syndrome, Refsum disease, Refsum disease - infantile, repetitive motion disorders, repetitive stress injury, Restless legs syndrome, retrovirus-associated myelopathy, Rett syndrome, Reye's syndrome, rheumatic encephalitis, rhythmic movement disorder, Riley-Day syndrome, Romberg syndrome, sacral nerve root cysts, saint vitus dance, Salivary gland disease, Sandhoff disease, Schilder's disease, schizencephaly, schizophrenia, Seitelberger disease, seizure disorder, semantic dementia, sensory integration dysfunction, septo-optic dysplasia, severe myoclonic epilepsy of infancy (SMEI), Shaken baby syndrome, shingles, Shy-Drager syndrome, Sjögren's syndrome, sleep apnea, sleeping sickness, snatiation, Sotos syndrome, spasticity, spina bifida, spinal cord infarction, spinal cord injury, spinal cord tumors, spinal muscular atrophy, spinocerebellar ataxia, spinocerebellar atrophy, spinocerebellar degeneration, Steele-Richardson-Olszewski syndrome, Stiff-Person syndrome, striatonigral degeneration, stroke, Sturge-Weber syndrome, subacute sclerosing panencephalitis, subcortical arteriosclerotic encephalopathy, SUNCT headache, superficial siderosis, swallowing disorders, sydenham's chorea, syncope, synesthesia, syphilitic spinal sclerosis, syringohydromyelia, syringomyelia, systemic lupus erythematosus, tabes dorsalis, tardive dyskinesia, tardive dysphrenia, tarlov cyst, Tarsal tunnel syndrome, Tay-Sachs disease, temporal arteritis, tetanus, Tethered spinal cord syndrome, Thomsen disease, thomsen's myotonia, Thoracic outlet syndrome, thyrotoxic myopathy, tic douloureux, todd's paralysis, Tourette syndrome, toxic encephalopathy, transient ischemic attack, transmissible spongiform encephalopathies, transverse myelitis, traumatic brain injury, tremor, trigeminal neuralgia, tropical spastic paraparesis, Troyer syndrome, trypanosomiasis, tuberous sclerosis, ubisiosis, uremia, vascular erectile tumor, vasculitis syndromes of the central and peripheral nervous systems, viliuisk encephalomyelitis (VE), Von economo's disease, Von Hippel-Lindau disease (VHL), Von recklinghausen's disease, Wallenberg's syndrome, Werdnig-Hoffman disease, Wernicke-Korsakoff syndrome, West syndrome, Whiplash, Whipple's disease, Williams syndrome, Wilson's disease, Wolman's disease, X-linked spinal and bulbar muscular atrophy, or Zellweger syndrome.

Methods of investigating neurological conditions are described, e.g., in U.S. Patent Application Publication No. 20120207726 and 2011018390.

### Mutations/Copy number variations

The methods described herein can be used to detect copy number variations. Diseases associated with copy number variations can include, for example, DiGeorge/velocardiofacial syndrome (22q11.2 deletion), Prader-Willi syndrome (15q11-q13 deletion), Williams-Beuren syndrome (7q11.23 deletion), Miller-Dieker syndrome (MDLS) (17p13.3 microdeletion), Smith-Magenis syndrome (SMS) (17p11.2 microdeletion), Neurofibromatosis Type 1 (NF1) (17q11.2 microdeletion), Phelan-McErmid Syndrome (22q13 deletion), Rett syndrome (loss-of-function mutations in MECp2 on chromosome Xq28), Merzbacher disease (CNV of PLP1), spinal muscular atrophy (SMA) (homozygous absence of telomerec SMN1 on chromosome 5q13), Potocki-Lupski Syndrome (PTLS, duplication of chromosome 17p.11.2). Additional copies of the PMP22 gene can be associated with Charcot-Marie-Tooth neuropathy type IA (CMT1A) and hereditary neuropathy with liability to pressure palsies (HNPP). The methods of detecting CNVs described herein can be used to diagnose CNV disorders described herein and in publications incorporated by reference. The disease can be a disease described in Lupski J. (2007) Nature Genetics 39: S43-S47.

The methods described herein can be used to detect fetal anueploides from a maternal sample, e.g., maternal blood sample, chorionic villus sample, or amniotic fluid. Aneuploides, e.g., fetal aneuploidies, can include, e.g., trisomy 13, trisomy 18, trisomy 21 (Down Syndrome), Klinefelter Syndrome (XXY), monosomy of one or more chromosomes (X chromosome monosomy, Turner's syndrome), trisomy X, trisomy of one or more chromosomes, tetrasomy or pentasomy of one or more chromosomes (e.g., XXXX, XXYY, XXXY, XYYY, XXXXX, XXXXY, XXXYY, XYYYY and XXYYY), triploidy (three of every chromosome, e.g. 69 chromosomes in humans), tetraploidy (four of every chromosome, e.g. 92 chromosomes in humans), and multiploidy. In some embodiments, an aneuploidy can be a segmental aneuploidy. Segmental aneuploidies can include, e.g., 1p36 duplication, dup(17)(p11.2p11.2) syndrome, Down syndrome, Pelizaeus-Merzbacher disease, dup(22)(q11.2q11.2) syndrome, and cat-eye syndrome. In some cases, an abnormal genotype, e.g., fetal genotype, is due to one or more deletions of sex or autosomal chromosomes, which can result in a condition such as Cri-du-chat syndrome, Wolf-Hirschhorn, Williams-Beuren syndrome, Charcot-Marie-Tooth disease, Hereditary neuropathy with liability to pressure palsies, Smith-Magenis syndrome, Neurofibromatosis, Alagille syndrome, Velocardiofacial syndrome, DiGeorge syndrome, Steroid sulfatase deficiency, Kallmann syndrome, Microphthalmia with linear skin defects, Adrenal hypoplasia, Glycerol kinase deficiency, Pelizaeus-Merzbacher disease, Testis-determining factor on Y, Azospermia (factor a), Azospermia (factor b), Azospermia (factor c), or 1p36 deletion. In some embodiments, a decrease in chromosomal number results in an XO syndrome. Methods for detecting fetal aneuploidy are described, e.g., in U.S. Patent No. 8293470.

Excessive genomic DNA copy number variation can be found in Li-Fraumeni cancer predisposition syndrome (Shlien et al. (2008) PNAS 105:11264-9). CNV is associated with malformation syndromes, including CHARGE (coloboma, heart anomaly, choanal atresia, retardation, gential, and ear anomalies), Peters-Plus, Pitt-Hopkins, and thrombocytopenia-absent radius syndrome (see e.g., Ropers HH (2007) Am J of Hum Genetics 81: 199-207). The relationship between copy number variations and cancer is described, e.g., in Shlien A. and Malkin D. (2009) Genome Med. 1(6): 62. Copy number variations are associated with, e.g., autism, schizophrenia, and idiopathic learning disability. *See e.g*., Sebat J., et al. (2007) Science 316: 445-9; Pinto J. et al. (2010) Nature 466: 368-72; Cook E.H. and Scherer S.W. (2008) Nature 455: 919-923.

Copy number variations can be associated with resistance of cancer patients to certain therapeutics. For example, amplification of thymidylate synthase can result in resistance to 5-fluorouracil treatment in metastatic colorectal cancer patients. *See* Wang et al. (2002) PNAS USA vol. 99, pp. 16156-61. Methods of determining CNVs are described, e.g., in PCT Application Publication No. WO2012/109500.

In some cases, the methods, compositions, or kits described herein are used to determine gene expression level (e.g., messenger RNA level). The expression level can be, for example, higher than normal, normal, or below normal. In some cases, the methods, compositions, and kits provided herein are used to determine if a sequence is mutated or wild-type, or has one or more mutations (e.g., a *de novo* mutation, nonsense mutation, missense mutation, silent mutation, frameshift mutation, insertion, substitution, point mutation, single nucleotide polymorphism (SNP), single nucleotide variant, *de novo* single nucleotide variant, deletion, rearrangement, amplification, chromosomal translocation, interstitial deletion, chromosomal inversion, loss of heterozygosity, loss of function, gain of function, dominant negative, or lethal).

Methods for analyzing nucleic acids, e.g., for detecting mutations, gene expression, or copy number variation, are described, e.g., in U.S. Patent Application Publication Nos. 20120252015, 2012021549, 20120214163, 20120225428, 20120245235, 20120252753, 20100196898, 20120270739, 20110171646, and U.S. Patent Nos. 8304194

### Additional assays

Nucleic acid used or produced using the methods, compositions, and kits described herein can be analyzed using one or more techniques. The one or more techniques can include array-based comparative genomic hybridization, detecting single nucleotide polymorphisms (SNPs) with arrays, expression profiling, DNA microarray, high-density oligonucleotide microarray, DNA sequencing, de novo DNA sequencing, 454 sequencing (Roche), pyrosequencing, Helicos True Single Molecule Sequencing, SOLiD™ sequencing (Applied Biosystems, Life Technologies), SOLEXA sequencing (Illumina sequencing), nanosequencing, chemical-sensitive field effect transistor (chemFET) array sequencing (Ion Torrent), ion semiconductor sequencing (Ion Torrent), DNA nanoball sequencing, nanopore sequencing, Pacific Biosciences SMRT sequencing, Genia Technologies nanopore single-molecule DNA sequencing, Oxford Nanopore single-molecule DNA sequencing, polony sequencing, copy number variation (CNV) analysis sequencing, small nucleotide polymorphism (SNP) analysis, mass spectrometry, tandem mass spectrometry, matrix-assisted laser desorption ionization time of flight mass spectrometry (MALDI-TOF MS), polymerase chain reaction (PCR), digital PCR (dPCR), quantitative PCR (Q-PCR), single marker qPCR, real-time PCR, nCounter Analysis (Nanostring technology), and gel electrophoresis. In some cases, RNA is analyzed by RNA sequencing or microarray.

In some cases, samples described herein can be analyzed by RT-PCR and by one or more other methods, including, e.g., immunohistochemistry (IHC), immunoctyochemistry (ICC), in-situ hybridization, fluorescent in-situ hybridization (FISH), chromogenic in-situ hybridization (CISH), silver in-situ hybridization (SISH), Far Western blotting, Western blotting, Southern blotting, SDS-PAGE, gel electrophoresis, and/or Northern blotting.

### Transcriptome analysis

The techniques described herein can be used to amplify the whole transcriptome (all the RNA molecules) of an organism, tissue, or cell. The RNA molecules can include messenger RNA, ribosomal RNA, transfer RNA, or other non-coding RNA. In some cases, the techniques described herein can be used to analyze only a portion of a transcriptome. In some cases, about, more than, less than, or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76 ,77, 78, 79, 80, 81, 82, 83, 84, 85, 86, 87, 88, 89, 90, 91, 92, 93, 94, 95, 96, 97, 98, 99, or 100% of a transcriptome of one or more cells or organisms under a given environmental condition is amplified. The transcriptome of a cell or organism can change based on the stage of development of the cell or organism or on environmental conditions. In some cases, the transcriptome of a cell or organism is analyzed or monitored over time. In some cases, the transcriptome from a subject is analyzed or monitored before and after treatment with a therapeutic agent, e.g., a drug.

In some cases, a transcriptome is analyzed by microarray or next-generation sequencing following RT-PCR.

In some cases, only messenger RNA from a transcriptome is analyzed using the methods, compositions, and kits provided herein. In some cases, messenger RNA is extracted from an RNA sample (e.g., by purification with an oligo-dT column or beads (e.g., magnetic beads), or coated plates). The coated plates can comprise locked nucleic acid (LNA) incorporated into oligo(dT) oligos. In some cases, messenger RNA is purified directly from crude samples.

In some cases, ribosomal RNA is removed, or purified, from an RNA sample. Extraction can involve use of organic extraction, silica column, and or magnetic beads.

In some cases, microRNA is removed, or purified, from an RNA sample.

Methods of transcriptome analysis are described, e.g., in U.S. Patent Application No. 20110189679 and U.S. Patent No. 7718403.

### Next generation sequencing

Next generation sequencing can be used injunction with the RT-PCR methods, compositions, and/or kits described herein (e.g., RNA-Seq) For example, cDNA produced by RT-PCR can be sequenced. In some cases, RNA used in an RT-PCR reaction, or a reference sample, is also sequenced.

Next-generation sequencing techniques include, for example, Helicos True Single Molecule Sequencing (tSMS) (Harris T.D. et al. (2008) Science 320:106-109); 454 sequencing (Roche) (Margulies, M. et al. 2005, Nature, 437, 376-380); SOLiD technology (Applied Biosystems); SOLEXA sequencing (Illumina); single molecule, real-time (SMRT™) technology of Pacific Biosciences; nanopore sequencing (Soni GV and Meller A. (2007) Clin Chem 53: 1996-2001); semiconductor sequencing (Ion Torrent; Personal Genome Machine); DNA nanoball sequencing; sequencing using technology from Dover Systems (Polonator), and technologies that do not require amplification or otherwise transform native DNA prior to sequencing (e.g., Pacific Biosciences and Helicos), such as nanopore-based strategies (e.g. Oxford Nanopore, Genia Technologies, and Nabsys).

The next generation sequencing technique can be 454 sequencing (Roche) (see e.g., Margulies, M et al. (2005) Nature 437: 376-380). 454 sequencing can involve two steps. In the first step, DNA can be sheared into fragments of approximately 300-800 base pairs, and the fragments can be blunt ended. Oligonucleotide adaptors can then ligated to the ends of the fragments. The adaptors can serve as sites for hybridizing primers for amplification and sequencing of the fragments. The fragments can be attached to DNA capture beads, e.g., streptavidin-coated beads using, e.g., Adaptor B, which can contain 5'-biotin tag. The fragments can be attached to DNA capture beads through hybridization. A single fragment can be captured per bead. The fragments attached to the beads can be PCR amplified within droplets of an oil-water emulsion. The result can be multiple copies of clonally amplified DNA fragments on each bead. The emulsion can be broken while the amplified fragments remain bound to their specific beads. In a second step, the beads can be captured in wells (e.g., pico-liter sized; PicoTiterPlate (PTP) device). The surface can be designed so that only one bead fits per well. The PTP device can be loaded into an instrument for sequencing. Pyrosequencing can be performed on each DNA fragment in parallel. Addition of one or more nucleotides can generate a light signal that can be recorded by a CCD camera in a sequencing instrument. The signal strength can be proportional to the number of nucleotides incorporated. Pyrosequencing can make use of pyrophosphate (PPi) which can be released upon nucleotide addition. PPi can be converted to ATP by ATP sulfurylase in the presence of adenosine 5' phosphosulfate. Luciferase can use ATP to convert luciferin to oxyluciferin, and this reaction can generate light that can be detected and analyzed. The 454 Sequencing system used can be GS FLX+ system or the GS Junior System.

The next generation sequencing technique can involve SOLiD technology (Applied Biosystems; Life Technologies). In SOLiD sequencing, genomic DNA can be sheared into fragments, and adaptors can be attached to the 5' and 3' ends of the fragments to generate a fragment library. Alternatively, internal adaptors can be introduced by ligating adaptors to the 5' and 3' ends of the fragments, circularizing the fragments, digesting the circularized fragment to generate an internal adaptor, and attaching adaptors to the 5' and 3' ends of the resulting fragments to generate a mate-paired library. Next, clonal bead populations can be prepared in microreactors containing beads, primers, template, and PCR components. Following PCR, the templates can be denatured and beads can be enriched to separate the beads with extended templates. Templates on the selected beads can be subjected to a 3' modification that permits bonding to a glass slide. A sequencing primer can bind to adaptor sequence. A set of four fluorescently labeled di-base probes can compete for ligation to the sequencing primer. Specificity of the di-base probe can be achieved by interrogating every first and second base in each ligation reaction. The sequence of a template can be determined by sequential hybridization and ligation of partially random oligonucleotides with a determined base (or pair of bases) that can be identified by a specific fluorophore. After a color is recorded, the ligated oligonucleotide can be cleaved and removed and the process can be then repeated. Following a series of ligation cycles, the extension product can be removed and the template can be reset with a primer complementary to the n-1 position for a second round of ligation cycles. Five rounds of primer reset can be completed for each sequence tag. Through the primer reset process, most of the bases can be interrogated in two independent ligation reactions by two different primers. Up to 99.99% accuracy can be achieved by sequencing with an additional primer using a multi-base encoding scheme.

In some cases, the next generation sequencing technique is SOLEXA sequencing (ILLUMINA sequencing). ILLUMINA sequencing can be based on the amplification of DNA on a solid surface using fold-back PCR and anchored primers. ILLUMINA sequencing can involve a library preparation step. Genomic DNA can be fragmented, and sheared ends can be repaired and adenylated. Adaptors can be added to the 5' and 3' ends of the fragments. The fragments can be size selected and purified. ILLUMINA sequence can comprise a cluster generation step. DNA fragments can be attached to the surface of flow cell channels by hybridizing to a lawn of oligonucleotides attached to the surface of the flow cell channel. The fragments can be extended and clonally amplified through bridge amplification to generate unique clusters. The fragments become double stranded, and the double stranded molecules can be denatured. Multiple cycles of the solid-phase amplification followed by denaturation can create several million clusters of approximately 1,000 copies of single-stranded DNA molecules of the same template in each channel of the flow cell. Reverse strands can be cleaved and washed away. Ends can be blocked, and primers can by hybridized to DNA templates. ILLUMINA sequencing can comprise a sequencing step. Hundreds of millions of clusters can be sequenced simultaneously. Primers, DNA polymerase and four fluorophore-labeled, reversibly terminating nucleotides can be used to perform sequential sequencing. All four bases can compete with each other for the template. After nucleotide incorporation, a laser can be used to excite the fluorophores, and an image can be captured and the identity of the first base can be recorded. The 3' terminators and fluorophores from each incorporated base can be removed and the incorporation, detection and identification steps can be repeated. A single base can be read each cycle. In some cases, a HiSeq system (e.g., HiSeq 2500, HiSeq 1500, HiSeq 2000, or HiSeq 1000) is used for sequencing. In some cases, a MiSeq personal sequencer is used. In some cases, a Genome Analyzer IIx is used.

The next generation sequencing technique can comprises real-time (SMRT™) technology by Pacific Biosciences. In SMRT, each of four DNA bases can be attached to one of four different fluorescent dyes. These dyes can be phospholinked. A single DNA polymerase can be immobilized with a single molecule of template single stranded DNA at the bottom of a zero-mode waveguide (ZMW). A ZMW can be a confinement structure which enables observation of incorporation of a single nucleotide by DNA polymerase against the background of fluorescent nucleotides that can rapidly diffuse in an out of the ZMW (in microseconds). It can take several milliseconds to incorporate a nucleotide into a growing strand. During this time, the fluorescent label can be excited and produce a fluorescent signal, and the fluorescent tag can be cleaved off. The ZMW can be illuminated from below. Attenuated light from an excitation beam can penetrate the lower 20-30 nm of each ZMW. A microscope with a detection limit of 20 zeptoliters (10⁻²¹ liters) can be created. The tiny detection volume can provide 1000-fold improvement in the reduction of background noise. Detection of the corresponding fluorescence of the dye can indicate which base was incorporated. The process can be repeated.

In some cases, the next generation sequencing is nanopore sequencing (*See e.g.,* Soni GV and Meller A. (2007) Clin Chem 53: 1996-2001). A nanopore can be a small hole, of the order of about one nanometer in diameter. Immersion of a nanopore in a conducting fluid and application of a potential across it can result in a slight electrical current due to conduction of ions through the nanopore. The amount of current which flows can be sensitive to the size of the nanopore. As a DNA molecule passes through a nanopore, each nucleotide on the DNA molecule can obstruct the nanopore to a different degree. Thus, the change in the current passing through the nanopore as the DNA molecule passes through the nanopore can represent a reading of the DNA sequence. The nanopore sequencing technology can be from Oxford Nanopore Technologies; e.g., a GridION system. A single nanopore can be inserted in a polymer membrane across the top of a microwell. Each microwell can have an electrode for individual sensing. The microwells can be fabricated into an array chip, with 100,000 or more microwells (e.g., more than 200,000, 300,000, 400,000, 500,000, 600,000, 700,000, 800,000, 900,000, or 1,000,000) per chip. An instrument (or node) can be used to analyze the chip. Data can be analyzed in real-time. One or more instruments can be operated at a time. The nanopore can be a protein nanopore, e.g., the protein alpha-hemolysin, a heptameric protein pore. The nanopore can be a solid-state nanopore made, e.g., a nanometer sized hole formed in a synthetic membrane (e.g., SiNx, or SiO₂). The nanopore can be a hybrid pore (e.g., an integration of a protein pore into a solid-state membrane). The nanopore can be a nanopore with an integrated sensors (e.g., tunneling electrode detectors, capacitive detectors, or graphene based nano-gap or edge state detectors (see e.g., Garaj et al. (2010) Nature vol. 67, doi:10.1038/nature09379)). A nanopore can be functionalized for analyzing a specific type of molecule (e.g., DNA, RNA, or protein). Nanopore sequencing can comprise "strand sequencing" in which intact DNA polymers can be passed through a protein nanopore with sequencing in real time as the DNA translocates the pore. An enzyme can separate strands of a double stranded DNA and feed a strand through a nanopore. The DNA can have a hairpin at one end, and the system can read both strands. In some cases, nanopore sequencing is "exonuclease sequencing" in which individual nucleotides can be cleaved from a DNA strand by a processive exonuclease, and the nucleotides can be passed through a protein nanopore. The nucleotides can transiently bind to a molecule in the pore (e.g., cyclodextran). A characteristic disruption in current can be used to identify bases.

Nanopore sequencing technology from GENIA can be used. An engineered protein pore can be embedded in a lipid bilayer membrane. "Active Control" technology can be used to enable efficient nanopore-membrane assembly and control of DNA movement through the channel. In some cases, the nanopore sequencing technology is from NABsys. Genomic DNA can be fragmented into strands of average length of about 100 kb. The 100kb fragments can be made single stranded and subsequently hybridized with a 6-mer probe. The genomic fragments with probes can be driven through a nanopore, which can create a current-versus-time tracing. The current tracing can provide the positions of the probes on each genomic fragment. The genomic fragments can be lined up to create a probe map for the genome. The process can be done in parallel for a library of probes. A genome-length probe map for each probe can be generated. Errors can be fixed with a process termed "moving window Sequencing By Hybridization (mwSBH)." In some cases, the nanopore sequencing technology is from IBM/Roche. An electron beam can be used to make a nanopore sized opening in a microchip. An electrical field can be used to pull or thread DNA through the nanopore. A DNA transistor device in the nanopore can comprise alternating nanometer sized layers of metal and dielectric. Discrete charges in the DNA backbone can get trapped by electrical fields inside the DNA nanopore. Turning off and on gate voltages can allow the DNA sequence to be read.

The next generation sequencing can comprise ion semiconductor sequencing (e.g., using technology from Life Technologies (Ion Torrent)). Ion semiconductor sequencing can take advantage of the fact that when a nucleotide is incorporated into a strand of DNA, an ion can be released. To perform ion semiconductor sequencing, a high density array of micromachined wells can be formed. Each well can hold a single DNA template. Beneath the well can be an ion sensitive layer, and beneath the ion sensitive layer can be an ion sensor. When a nucleotide is added to a DNA, H+ can be released, which can be measured as a change in pH. The H+ ion can be converted to voltage and recorded by the semiconductor sensor. An array chip can be sequentially flooded with one nucleotide after another. No scanning, light, or cameras can be required. In some cases, an IONPROTON™ Sequencer is used to sequence nucleic acid. In some cases, an IONPGM™ Sequencer is used.

The next generation sequencing can comprise DNA nanoball sequencing (as performed, e.g., by Complete Genomics; see e.g., Drmanac et al. (2010) Science 327: 78-81). DNA can be isolated, fragmented, and size selected. For example, DNA can be fragmented (e.g., by sonication) to a mean length of about 500 bp. Adaptors (Ad1) can be attached to the ends of the fragments. The adaptors can be used to hybridize to anchors for sequencing reactions. DNA with adaptors bound to each end can be PCR amplified. The adaptor sequences can be modified so that complementary single strand ends bind to each other forming circular DNA. The DNA can be methylated to protect it from cleavage by a type IIS restriction enzyme used in a subsequent step. An adaptor (e.g., the right adaptor) can have a restriction recognition site, and the restriction recognition site can remain non-methylated. The non-methylated restriction recognition site in the adaptor can be recognized by a restriction enzyme (e.g., Acul), and the DNA can be cleaved by AcuI 13 bp to the right of the right adaptor to form linear double stranded DNA. A second round of right and left adaptors (Ad2) can be ligated onto either end of the linear DNA, and all DNA with both adapters bound can be PCR amplified (e.g., by PCR). Ad2 sequences can be modified to allow them to bind each other and form circular DNA. The DNA can be methylated, but a restriction enzyme recognition site can remain non-methylated on the left Ad1 adapter. A restriction enzyme (e.g., Acul) can be applied, and the DNA can be cleaved 13 bp to the left of the Ad1 to form a linear DNA fragment. A third round of right and left adaptor (Ad3) can be ligated to the right and left flank of the linear DNA, and the resulting fragment can be PCR amplified. The adaptors can be modified so that they can bind to each other and form circular DNA. A type III restriction enzyme (e.g., EcoP15) can be added; EcoP15 can cleave the DNA 26 bp to the left of Ad3 and 26 bp to the right of Ad2. This cleavage can remove a large segment of DNA and linearize the DNA once again. A fourth round of right and left adaptors (Ad4) can be ligated to the DNA, the DNA can be amplified (e.g., by PCR), and modified so that they bind each other and form the completed circular DNA template. Rolling circle replication (e.g., using Phi 29 DNA polymerase) can be used to amplify small fragments of DNA. The four adaptor sequences can contain palindromic sequences that can hybridize and a single strand can fold onto itself to form a DNA nanoball (DNB™) which can be approximately 200-300 nanometers in diameter on average. A DNA nanoball can be attached (e.g., by adsorption) to a microarray (sequencing flowcell). The flow cell can be a silicon wafer coated with silicon dioxide, titanium and hexamehtyldisilazane (HMDS) and a photoresist material. Sequencing can be performed by unchained sequencing by ligating fluorescent probes to the DNA. The color of the fluorescence of an interrogated position can be visualized by a high resolution camera. The identity of nucleotide sequences between adaptor sequences can be determined.

In some cases, the next generation sequencing technique is Helicos True Single Molecule Sequencing (tSMS) (see e.g., Harris T. D. et al. (2008) Science 320:106-109). In the tSMS technique, a DNA sample can be cleaved into strands of approximately 100 to 200 nucleotides, and a polyA sequence can be added to the 3' end of each DNA strand. Each strand can be labeled by the addition of a fluorescently labeled adenosine nucleotide. The DNA strands can then be hybridized to a flow cell, which can contain millions of oligo-T capture sites immobilized to the flow cell surface. The templates can be at a density of about 100 million templates/cm². The flow cell can then be loaded into an instrument, e.g., HELISCOPE™ sequencer, and a laser can illuminate the surface of the flow cell, revealing the position of each template. A CCD camera can map the position of the templates on the flow cell surface. The template fluorescent label can then be cleaved and washed away. The sequencing reaction can begin by introducing a DNA polymerase and a fluorescently labeled nucleotide. The oligo-T nucleic acid can serve as a primer. The DNA polymerase can incorporate the labeled nucleotides to the primer in a template directed manner. The DNA polymerase and unincorporated nucleotides can be removed. The templates that have directed incorporation of the fluorescently labeled nucleotide can be detected by imaging the flow cell surface. After imaging, a cleavage step can remove the fluorescent label, and the process can be repeated with other fluorescently labeled nucleotides until a desired read length is achieved. Sequence information can be collected with each nucleotide addition step. The sequencing can be asynchronous. The sequencing can comprise at least 1 billion bases per day or per hour.

In some cases, the sequencing technique can comprise paired-end sequencing in which both the forward and reverse template strand can be sequenced. In some cases, the sequencing technique can comprise mate pair library sequencing. In mate pair library sequencing, DNA can be fragments, and 2-5 kb fragments can be end-repaired (e.g., with biotin labeled dNTPs). The DNA fragments can be circularized, and non-circularized DNA can be removed by digestion. Circular DNA can be fragmented and purified (e.g., using the biotin labels). Purified fragments can be end-repaired and ligated to sequencing adaptors.

In some cases, a sequence read is about, more than, less than, or at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 525, 550, 575, 600, 625, 650, 675, 700, 725, 750, 775, 800, 825, 850, 875, 900, 925, 950, 975, 1000, 1100, 1200, 1300, 1400, 1500, 1600, 1700, 1800, 1900, 2000, 2100, 2200, 2300, 2400, 2500, 2600, 2700, 2800, 2900, or 3000 bases. In some cases, a sequence read is about 10 to about 50 bases, about 10 to about 100 bases, about 10 to about 200 bases, about 10 to about 300 bases, about 10 to about 400 bases, about 10 to about 500 bases, about 10 to about 600 bases, about 10 to about 700 bases, about 10 to about 800 bases, about 10 to about 900 bases, about 10 to about 1000 bases, about 10 to about 1500 bases, about 10 to about 2000 bases, about 50 to about 100 bases, about 50 to about 150 bases, about 50 to about 200 bases, about 50 to about 500 bases, about 50 to about 1000 bases, about 100 to about 200 bases, about 100 to about 300 bases, about 100 to about 400 bases, about 100 to about 500 bases, about 100 to about 600 bases, about 100 to about 700 bases, about 100 to about 800 bases, about 100 to about 900 bases, or about 100 to about 1000 bases.

The number of sequence reads from a sample can be about, more than, less than, or at least 100, 1000, 5,000, 10,000, 20,000, 30,000, 40,000, 50,000, 60,000, 70,000, 80,000, 90,000, 100,000, 200,000, 300,000, 400,000, 500,000, 600,000, 700,000, 800,000, 900,000, 1,000,000, 2,000,000, 3,000,000, 4,000,000, 5,000,000, 6,000,000, 7,000,000, 8,000,000, 9,000,000, or 10,000,000.

The depth of sequencing of a sample can be about, more than, less than, or at least 1x, 2x, 3x,4x, 5x, 6x, 7x, 8x, 9x, 10x, 11x, 12x, 13x, 14x, 15x, 16x, 17x, 18x, 19x, 20x, 21x, 22x, 23x, 24x, 25x, 26x, 27x, 28x, 29x, 30x, 31x, 32x, 33x, 34x, 35x, 36x, 37x, 38x, 39x, 40x, 41x, 42x, 43x, 44x, 45x, 46x, 47x, 48x, 49x, 50x, 51x, 52x, 53x, 54x, 55x, 56x, 57x, 58x, 59x, 60x, 61x, 62x, 63x, 64x, 65x, 66x, 67x, 68x, 69x, 70x, 71x, 72x, 73x, 74x, 75x, 76x, 77x, 78x, 79x, 80x, 81x, 82x, 83x, 84x, 85x, 86x, 87x, 88x, 89x, 90x, 91x, 92x, 93x, 94x, 95x, 96x, 97x, 98x, 99x, 100x, 110x, 120x, 130x, 140x, 150x, 160x, 170x, 180x, 190x, 200x, 300x,400x, 500x, 600x, 700x, 800x, 900x, 1000x, 1500x,2000x,2500x, 3000x, 3500x, 4000x, 4500x, 5000x, 5500x, 6000x, 6500x, 7000x, 7500x, 8000x, 8500x, 9000x, 9500x, or 10,000x. The depth of sequencing of a sample can about 1x to about 5x, about 1x to about 10x, about 1x to about 20x, about 5x to about 10x, about 5x to about 20x, about 5x to about 30x, about 10x to about 20x, about 10x to about 25x, about 10x to about 30x, about 10x to about 40x, about 30x to about 100x, about 100x to about 200x, about 100x to about 500x, about 500x to about 1000x, about 1000x, to about 2000x, about 1000x to about 5000x, or about 5000x to about 10,000x. Depth of sequencing can be the number of times a sequence (e.g., a genome) is sequenced. In some cases, the Lander/Waterman equation is used for computing coverage. The general equation can be: C = LN/G, where C = coverage; G = haploid genome length; L = read length; and N = number of reads.

In some cases, different barcodes can be added to polynucleotides in different samples (e.g., by using primers or adaptors), and the different samples can be pooled and analyzed in a multiplexed assay. The barcode can allow the determination of the sample from which a polynucleotide originated. In some cases, nucleic acid derived from at least, more than, less than, or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200, 300, 400, 500, 600, 700, 800, 900, or 1000 samples is pooled. In some cases, partitions, such as droplets, comprising adaptors with barcodes are merged with droplets comprising nucleic acid to introduce a barcode on the nucleic acid. Methods for barcoding nucleic acid samples using droplets are described, e.g., in PCT Application Publication No. WO/2012/149042.

### Computers

The methods described herein can be implemented by one or more computer systems. Computer systems can include various combinations of a central processor or other processing device, an internal communication bus, various types of memory or storage media (RAM, ROM, EEPROM, cache memory, disk drives, etc.) for code and data storage, and one or more network interface cards or ports for communication purposes. The methods described herein can include or be implemented in software code, which can run on such computer systems or other systems. For example, the software code can be executable by a computer system, for example, that functions as the storage server or proxy server, and/or that functions as a user's terminal device. During operation the code can be stored within the computer system. At other times, the code can be stored at other locations and/or transmitted for loading into the appropriate computer system. Execution of the code by a processor of the computer system can enable the computer system to implement the methods described herein.

In some cases, a non-transitory computer readable medium is provided, wherein the non-transitory computer readable medium has stored thereon sequences of instructions which, when executed by a computer system, cause the computer to perform methods described herein. Computer readable media can include, e.g., a hard disk, diskette, random-access memory ("RAM"), read-only memory ("ROM"), programmable read-only memory ("PROM"), erasable programmable read-only memory ("EPROM"), electrically erasable programmable read-only memory ("EEPROM"), compact disk read-only memory ("CD-ROM"), CD±R, CD±RW, DVD, DVD±RW, DVD±R, DVD-RAM, HD DVD, HD DVDR, HD DVD±RW, HD DVD±RAM, Blu-ray Disc, optical or magnetic storage medium, paper tape, punch cards, optical mark sheets or any other device that is capable of providing data or executable instructions that can be accessed by a processing system. Computer readable medium are described, e.g., in U.S. Patent No. 7783072.

A computer can be in communication with a device, e.g., a thermocycler or a device for performing droplet digital PCR. A computer can be connected to the Internet through a wired or wireless connection.

In some cases, a health care provider or subject sends a sample to a service provider that analyzes the sample using the methods, compositions, or kits described herein. In some cases, a computer is used to transmit results of an RT-PCR reaction to a subject. In some cases, the subject is a patient. In some cases, a computer is used to transmit results of an RT-PCR reaction to a healthcare provider, e.g., a physician, or to an insurance company. In some cases, a computer is used to generate a report comprising results of one or more RT-PCR reactions and/or additional assays.

### IV. Examples

### Example 1

The following is a protocol to run a full plate of one-step reverse transcription PCR using a single gene target using Bio-Rad's One-Step RT-ddPCR™ Kit for Probes on Bio-Rad's QX100™ Droplet Digital™ PCR System.

### Materials

| | | |
|---|---|---|
| 2X One-Step RT-ddPCR Supermix Reagents | 1 tube | 1,000 µL per tube |
| 25 mM Manganese Acetate Solution | 1 tube | 1,000 µL per tube |

20X Gene Target Primer/Probe Mix
RNA sample (range from 5 ng to 0.5 pg total RNA per reaction)
RNase-, DNase-free, Molecular Bio-Grade water

### Required Equipmen

Standard bench equipment - pipettes, plate centrifuge
Thermal Cycler for droplet PCR
Bio-Rad QX100™ Droplet Generator
Bio-Rad QX100™ Droplet Reader

### Setting up the RT Reaction

• Prepare the RNA samples and the desired concentrations before setting up the RT Reaction Mix and keep them on ice.
• Allow the reagents to thaw on ice or at 4 °C.
• Prepare 2 mL of reaction mix as described in the table below. This should be enough for 96 reactions at 20 µL each. Keep the reaction mix on ice until ready to generate droplets.
• As soon as all the reaction mixes are ready, allow the reaction tubes to stabilize at room temperature for about 3 minutes before loading into the DG8 cartridge.
• Dispense 20 µL of the sample mix into the DG8 cartridge.

| Reaction Component | Volume per Reaction (µL) | Volume per 100 Reactions (µL) |
|---|---|---|
| 2X One-Step RT-ddPCR Supermix Reagents | 10 | 1000 |
| 25 mM Manganese Acetate | 0.8 | 80 |
| 20X Gene Target Primer/Probe Mix | 1 | 100 |
| RNA Sample | Variable | Variable |
| Water | Variable | Variable |
| Total | 20 | 2000 |

### ddPCR Plate Layout

- After each droplet generation run (8 samples per run), dispense the droplets into an 8 well column on the PCR plate.
- The PCR plate containing the droplets does not need to be on ice while the rest of the sample droplets are being prepared. Suitable plates are Eppendorf skirted plates.
- Seal the plate with aluminum foil seal on the Foil Heat Sealer. Rotate the plate 180 °C and repeat the process twice to ensure proper sealing.

### PCR Amplification Parameters

Load the sealed plate on an Eppendorf or BioRad thermal cycler using the protocol below.

| | RT Step | Polymeras e Activation | PCR | | Enzym e Heat Kill | (Optional ) |
|---|---|---|---|---|---|---|
| | Hol d | Hold | 40 Cycles | | Hold | Hold |
| | | | Denatur e | Anneal/Exten d | | |
| Temperatur e | 60 °C | 95 °C | 94 °C | 60 °C | 98 °C | 4 °C |
| Time | 30 min | 5 min | 30 sec | 1 min | 10 min | forever |

Standard settings
50% ramps (∼2 - 3 °C/sec)

### Example 2

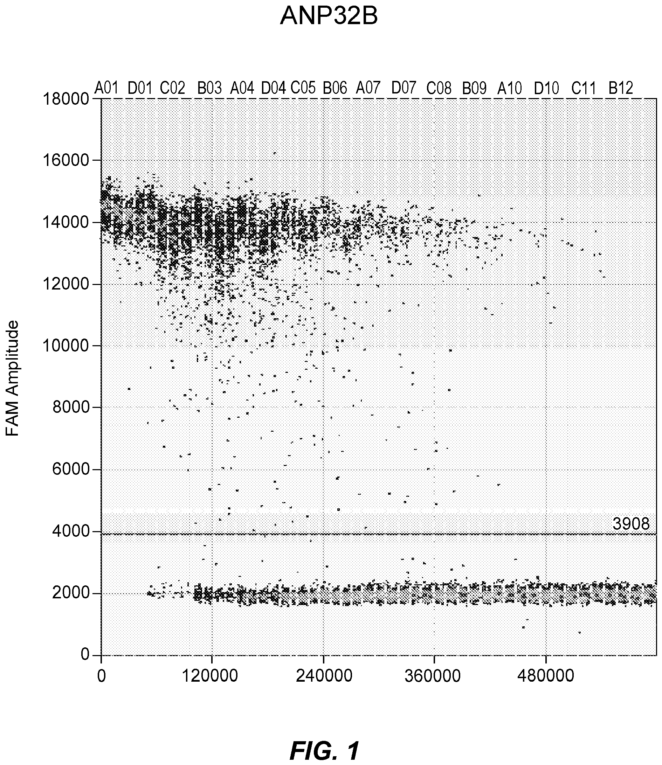

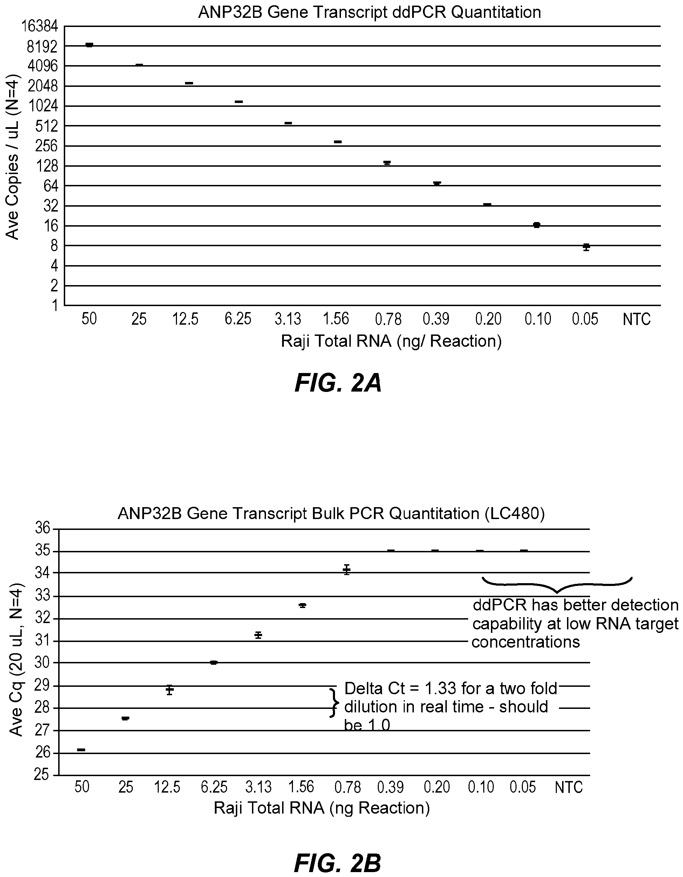

Two-fold serial dilutions of the Raji Total RNA template were prepared and reverse-transcribed and amplified using the One-Step RT-ddPCR Supermix. Figures 1 and 2A are quantification of Acidic leucine-rich nuclear phosphoprotein 32 family member B (*ANP32B*) gene transcript by droplet PCR. Figure 2B is quantification of ANP32B gene transcript by real-time qRT-PCR

The ddPCR™ Hot-start 1 Step RT-PCR Master Mix has a 3 log dynamic range and linear quantitation. See Fig. 2A

In real time the delta Ct in two fold dilutions is 1.33 (not 1.0), indicating a change in RT efficiency with concentration. FIG. 2B

In RT-ddPCR, the reported concentration is exactly two fold and correlates with two-fold dilution of input RNA template showing that RT efficiency is preserved across target concentrations. FIG. 2A

### Example 3

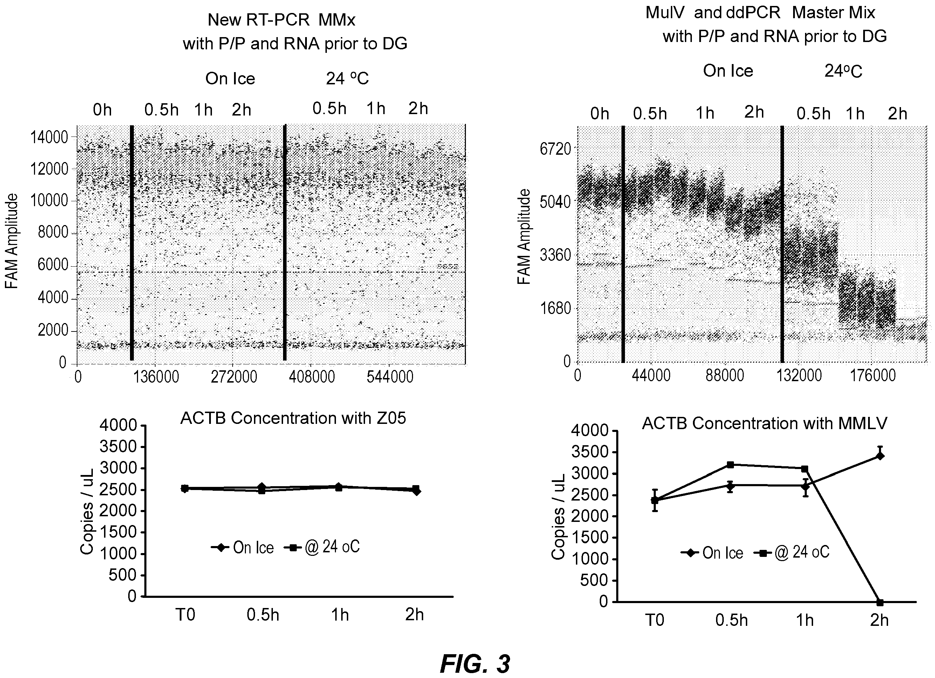

Figure 3 illustrates detection of Beta-Actin gene transcript using the One-Step RT-ddPCR™ Supermix and the standard RT mix containing MMLV and magnesium in a droplet digital PCR system. The RT mix containing all the reaction components including 1.6 ng of Raji Total RNA were incubated on ice or at 24 °C mimicking the workflow conditions before the droplets are loaded on the thermocycler.

No variation on reported detectable RNA concentration exists despite variations in workflow conditions prior to the amplification of the droplets with the ddPCR™ Hot-start 1 Step RT-PCR Master Mix.

### Example 4

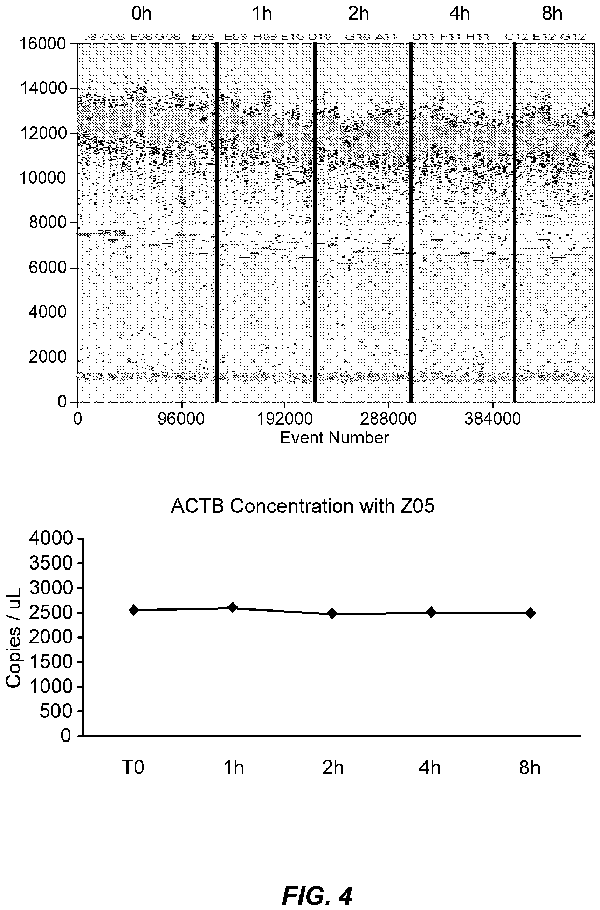

Stability of RT droplets on ice prior to RT-PCR. Figure 4 shows the stability of the droplets which contain the RT reaction after droplet generation and prior to RT-PCR. The uniform concentration reported indicates no pre-mature enzyme activation loss of enzyme activity. Upper chart shows droplet fluorescence amplitude (Y-axis) by droplet count and incubation time (X-axis). Negative droplets have an amplitude of approximately 1200 rfu and Positive droplets with an amplitude of approximately 12,000 rfu. The bottom chart shows amplitude does not change with workflow variability and the reaction mix is stable.

The lower chart shows average concentration of the RNA target as copies/uL versus workflow variability from 0 to 8 hours. This chart indicates that the RNA in the reaction mix is stable with various workflow times.

### Example 5

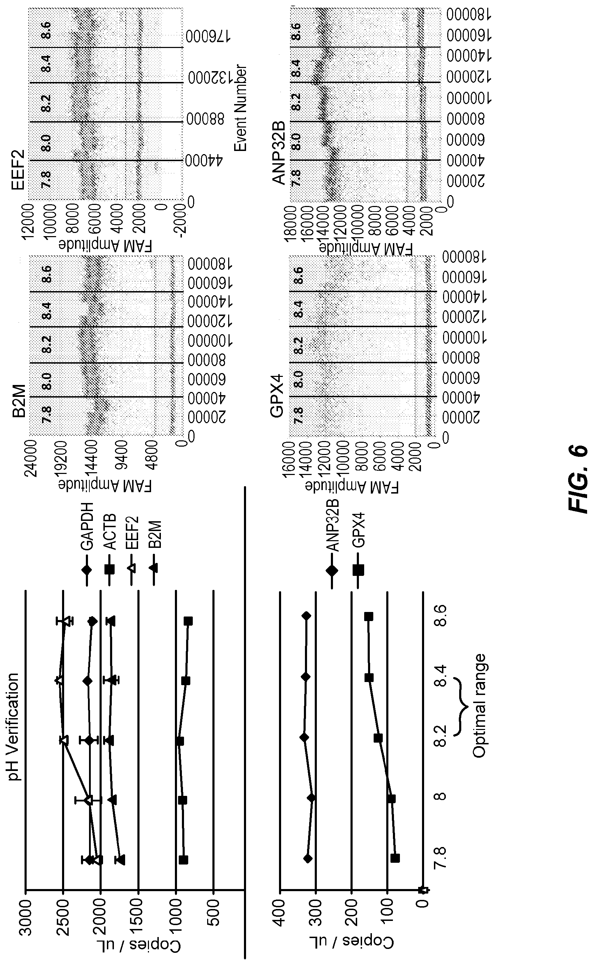

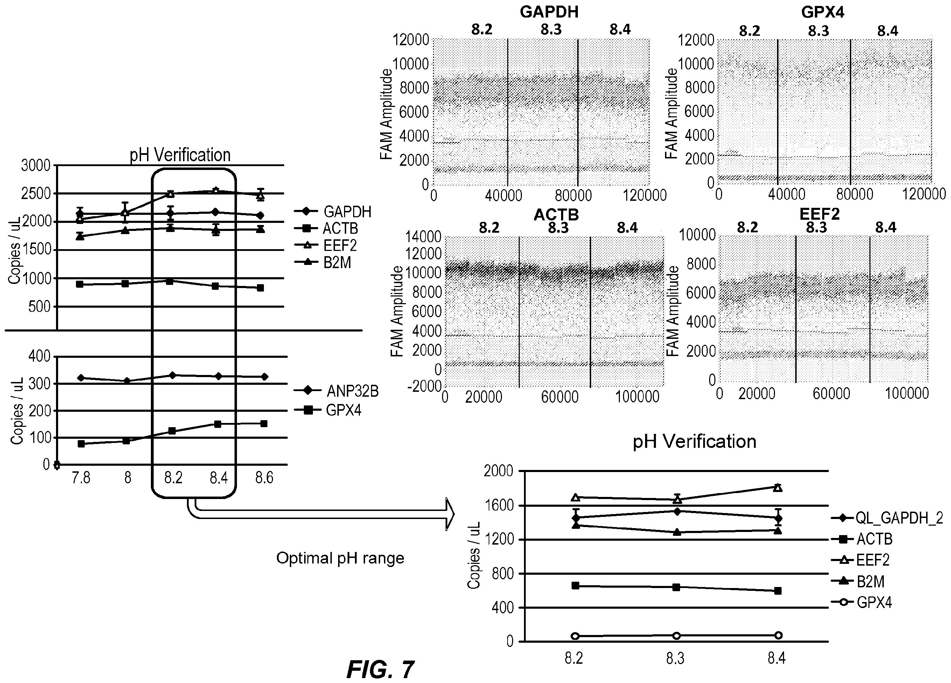

**FIG. 6** and **FIG. 7** show the pH optimization for One-Step RT-ddPCR™ Supermix. A final concentration of 50 mM of Tris was added to each solution and then HCl was added to adjust the pH to 7.8, 8.0, 8.2, 8.4 and 8.6.

**FIG. 7** shows amplitude (y-axis) versus droplet count/pH (x-axis) plots, and shows concentration (y-axis) versus pH (x-axis) plots. The amplitude plots indicate that the reaction mix is tolerant to a broad range of pH conditions and the concentration plots show that the concentration reporting is tolerant to a broad range of pH but most optimium between pH 8.2 and 8.4.

### Example 6

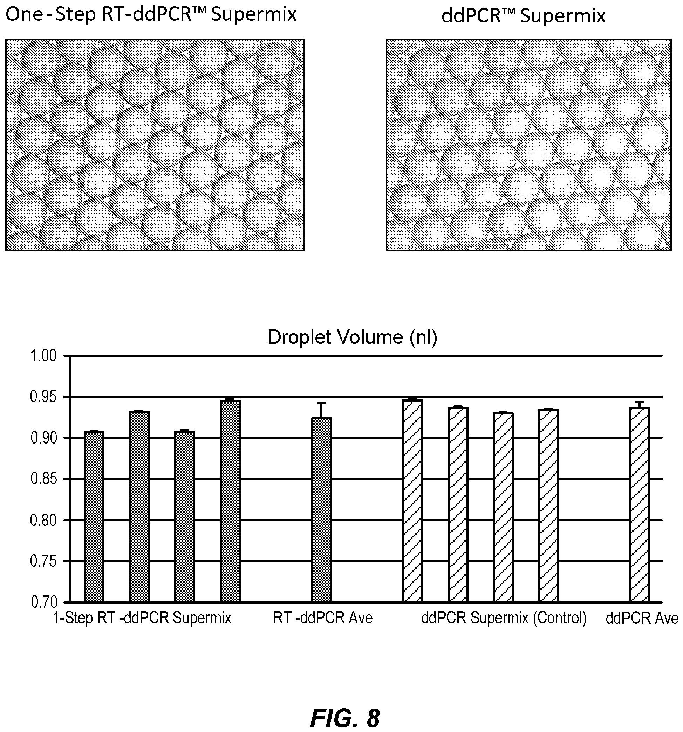

Sizes of RT Mix droplets are similar to that of current ddPCR Master Mix. **FIG. 8** shows the droplets generated with One-Step RT-ddPCR™ Supermix prior to RT-PCR.

## Claims

1. A method of performing reverse transcription and amplification, the method comprising:
(a) providing a first solution including
(i) a buffer,
(ii) a magnesium salt,
(iii) dNTPs comprising dATP, dCTP, dGTP, and dTTP, or analogs thereof,
(iv) an enzyme comprising reverse transcriptase activity, and
(v) an enzyme comprising DNA-dependent DNA polymerase activity;
(b) mixing the first solution with a second solution comprising manganese;
(c) forming emulsion droplets containing the first solution mixed with the second solution;
(d) reverse-transcribing RNA in the droplets with the first enzyme to form DNA; and
(e) amplifying a target sequence of the DNA in the emulsion droplets with the second enzyme;
wherein the molar ratio between the magnesium and the dNTPs in the first solution is about 1:1.

2. The method of claim 1, wherein the first solution comprises a monovalent cation.

3. The method of claim 1 or claim 2, wherein the first solution comprises an oligonucleotide to prime reverse transcription.

4. The method of any of claims 1 to 3, wherein the first solution comprises a pair of primers for amplifying the target sequence.

5. The method of any of claims 1 to 4, wherein the enzyme comprising reverse transcriptase activity and the enzyme comprising DNA-dependent DNA polymerase activity are the same enzyme.

6. The method of any of claims 1 to 5, wherein the dATP, dCTP, dGTP, and dTTP, or analogs thereof are collectively present in the droplets at a first concentration, wherein magnesium is present in the droplets at a second concentration, and wherein the ratio of the first concentration to the second concentration is about 1:1.

7. The method of any of claims 1 to 6, wherein magnesium is present in the droplets at a concentration of about 1 mM.

8. The method of any of claims 1 to 7, wherein manganese is present in the droplets at a concentration of from 0.5 mM to 5 mM.

9. The method of claim 8, wherein manganese is present in the droplets at a concentration of about 1 mM.

10. The method of any of claims 1 to 9, wherein manganese and magnesium are present in the droplets at a concentration ratio of from about 1:4 to about 3:1.

11. The method of claim 10, wherein the concentration ratio is about 1:1.

12. The method of any of claims 1 to 11, wherein the step of amplifying a target sequence includes a step of thermocycling the droplets.

13. The method of any of claims 1 to 12, wherein the droplets have a volume that is between about 1 pL and about 100 nL.

14. The method of any of claims 1 to 13, wherein the first solution comprises a magnesium salt selected from the group consisting of MgCl2, MgS0₄, and Mg(OAc)₂.

15. The method of any of claims 1 to 14, wherein the first solution comprises a dNTP analog.

## Patentansprüche

1. Verfahren zum Durchführen von umgekehrter Transkription und Amplifikation, wobei das Verfahren Folgendes umfasst:
(a) Bereitstellen einer ersten Lösung, Folgendes enthaltend:
(i) einen Puffer,
(ii) ein Magnesiumsalz,
(iii)dNTPs, dATP, dCTP, dGTP und dTTP oder Analoga davon umfassend,
(iv)ein Enzym, umgekehrte Transkriptase-Aktivität umfassend, und
(v) ein Enzym, DNA-abhängige DNA-Polymerase-Aktivität umfassend;
(b) Mischen der ersten Lösung mit einer zweiten Lösung, die Mangan umfasst;
(c) Ausbilden von Emulsionströpfchen, die die mit der zweiten Lösung gemischte erste Lösung enthalten;
(d) umgekehrtes Transkribieren von RNA in den Tröpfchen mit dem ersten Enzym, um DNA auszubilden; und
(e) Verstärken einer Zielsequenz der DNA in den Emulsionströpfchen mit dem zweiten Enzym;
wobei das Molverhältnis zwischen dem Magnesium und den dNTPs in der ersten Lösung etwa 1:1 ist.

2. Verfahren nach Anspruch 1, wobei die erste Lösung ein monovalentes Kation umfasst.

3. Verfahren nach Anspruch 1 oder 2, wobei die erste Lösung ein Oligonukleotid zum Vorbereiten von umgekehrter Transkription umfasst.

4. Verfahren nach einem der Ansprüche 1 bis 3, wobei die erste Lösung ein Paar von Primern zum Verstärken der Zielsequenz umfasst.

5. Verfahren nach einem der Ansprüche 1 bis 4, wobei das Enzym, das umgekehrte Transkriptase-Aktivität umfasst, und das Enzym, das DNA-abhängige DNA-Polymerase-Aktivität umfasst, dasselbe Enzym sind.

6. Verfahren nach einem der Ansprüche 1 bis 5, wobei das dATP, dCTP, dGTP und dTTP oder Analoga davon in den Tröpfchen in einer ersten Konzentration gesammelt vorhanden sind, wobei Magnesium in den Tröpfchen in einer zweiten Konzentration vorhanden ist und wobei das Verhältnis der ersten Konzentration zu der zweiten Konzentration etwa 1:1 ist.

7. Verfahren nach einem der Ansprüche 1 bis 6, wobei Magnesium in den Tröpfchen in einer Konzentration von etwa 1 mM vorhanden ist.

8. Verfahren nach einem der Ansprüche 1 bis 7, wobei Mangan in den Tröpfchen in einer Konzentration von 0,5 mM bis 5 mM vorhanden ist.

9. Verfahren nach Anspruch 8, wobei Mangan in den Tröpfchen in einer Konzentration von etwa 1 mM vorhanden ist.

10. Verfahren nach einem der Ansprüche 1 bis 9, wobei Mangan und Magnesium in den Tröpfchen in einem Konzentrationsverhältnis von etwa 1:4 bis etwa 3:1 vorhanden sind.

11. Verfahren nach Anspruch 10, wobei das Konzentrationsverhältnis etwa 1:1 ist.

12. Verfahren nach einem der Ansprüche 1 bis 11, wobei der Schritt des Verstärkens einer Zielsequenz den Schritt des thermischen zyklischen Durchlaufens der Tröpfchen enthält.

13. Verfahren nach einem der Ansprüche 1 bis 12, wobei die Tröpfchen ein Volumen aufweisen, das zwischen etwa 1 pL und etwa 100 nL liegt.

14. Verfahren nach einem der Ansprüche 1 bis 13, wobei die erste Lösung ein Magnesiumsalz umfasst, ausgewählt aus der Gruppe bestehend aus MgCl2, MgS0₄ und Mg(OAc)2.

15. Verfahren nach einem der Ansprüche 1 bis 14, wobei die erste Lösung ein dNTP-Analog umfasst.

## Revendications

1. Procédé de réalisation d'une transcription inverse et d'une amplification, le procédé comprenant :
(a) la fourniture d'une première solution comportant
(i) un tampon,
(ii) un sel de magnésium,
(iii) des dNTP comprenant dATP, dCTP, dGTP, et dTTP, ou leurs analogues,
(iv) une enzyme comprenant une activité de transcriptase inverse, et
(v) une enzyme comprenant une activité d'ADN polymérase dépendant de l'ADN ;
(b) le mélange de la première solution avec une seconde solution comprenant du manganèse ;
(c) la formation de gouttelettes d'émulsion contenant la première solution mélangée avec la seconde solution ;
(d) la transcription inverse de l'ARN dans les gouttelettes avec la première enzyme pour former de l'ADN ; et
(e) l'amplification d'une séquence cible de l'ADN dans les gouttelettes d'émulsion avec la seconde enzyme ;
dans lequel le rapport molaire entre le magnésium et les dNTP dans la première solution est d'environ 1:1.

2. Procédé selon la revendication 1, dans lequel la première solution comprend un cation monovalent.

3. Procédé selon la revendication 1 ou la revendication 2, dans lequel la première solution comprend un oligonucléotide pour amorcer une transcription inverse.

4. Procédé selon l'une quelconque des revendications 1 à 3, dans lequel la première solution comprend une paire d'amorces pour amplifier la séquence cible.

5. Procédé selon l'une quelconque des revendications 1 à 4, dans lequel l'enzyme comprenant une activité de transcription inverse et l'enzyme comprenant une activité d'ADN polymérase dépendant de l'ADN sont la même enzyme.

6. Procédé selon l'une quelconque des revendications 1 à 5, dans lequel les dATP, dCTP, dGTP, et dTTP, ou leurs analogues sont collectivement présents dans les gouttelettes à une première concentration, dans lequel du magnésium est présent dans les gouttelettes à une seconde concentration, et dans lequel le rapport entre la première concentration et la seconde concentration est d'environ 1:1.

7. Procédé selon l'une quelconque des revendications 1 à 6, dans lequel le magnésium est présent dans les gouttelettes à une concentration d'environ 1 mM.

8. Procédé selon l'une quelconque des revendications 1 à 7, dans lequel du manganèse est présent dans les gouttelettes à une concentration allant de 0,5 mM à 5 mM.

9. Procédé selon la revendication 8, dans lequel du manganèse est présent dans les gouttelettes à une concentration d'environ 1 mM.

10. Procédé selon l'une quelconque des revendications 1 à 9, dans lequel du manganèse et du magnésium sont présents dans les gouttelettes à un rapport de concentration allant d'environ 1:4 à environ 3:1.

11. Procédé selon la revendication 10, dans lequel le rapport de concentration est d'environ 1:1.

12. Procédé selon l'une quelconque des revendications 1 à 11, dans lequel l'étape d'amplification d'une séquence cible inclut une étape de thermocyclage des gouttelettes.

13. Procédé selon l'une quelconque des revendications 1 à 12, dans lequel les gouttelettes ont un volume qui est compris entre environ 1 pL et environ 100 nL.

14. Procédé selon l'une quelconque des revendications 1 à 13, dans lequel la première solution comprend un sel de magnésium choisi dans le groupe consistant en MgCl2, MgS0₄, et Mg(OAc)2.

15. Procédé selon l'une quelconque des revendications 1 à 14, dans lequel la première solution comprend un analogue de dNTP.
